# EUROPEAN PATENT APPLICATION

(11) **EP 4 098 252 A1**
(43) Date of publication of application: **07.12.2022**
(21) Application number: 21386032.3
(22) Date of filing: 02.06.2021
(51) Int. Cl.: A61K 9/16, A61K 31/121, A61K 31/122, A61K 31/167, A61P 43/00

(54) **POLY AMPHIPHILIC COMPLEXES FOR THE DELIVERY OF A HYDROPHOBIC ACTIVE AGENT, COMPOSITIONS AND METHODS**

(71) Applicant: Université de Liège, 4000 Liège (BE); Fundacio Institut de Bioenginyeria de Catalunya, 08028 Barcelona (ES); Barcelona Institute For Global Health, 08036 Barcelona (ES); Foundation For Research And Technology Hellas, 70013 Heraklion, Crete (GR)
(72) Inventor: Grandfils, Christian, 4000 Liège (BE); Sevrin, Chantal, 4000 Liège (BE); Fernàndez Busquets, Xavier, ES08036 Barcelona (ES); Siden-Kiamos, Inga, GR-70013 Heraklion, Crete (GR); Vontas, John, GR-70013 Heraklion, Crete (GR)
(74) Representative: Peel, James Peter

(57) **Abstract**

The invention provides a complex comprising at least one hydrophobic active agent, an ionic polymer comprising a repetitive unit of formula (I): 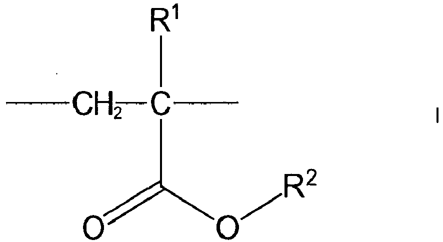 wherein R¹ represents a hydrogen atom or a straight or branched chain alkyl group, preferably a straight or branched chain alkyl group comprising from 1 to 6 carbon atoms, for example a methyl group; R² represents a straight or branched chain alkyl group which is substituted by a group which may have a positive charge at a physiological pH; a complex for use in a method of medical treatment; a pharmaceutical composition; and a method of preparing a complex according to the invention which method comprises the steps of:
(a) dissolving the hydrophobic active agent and the ionic polymer in one or more non-aqueous solvents to form the complex wherein the one or more non-aqueous solvents are miscible with water; and
(b) progressively replacing the one or more non-aqueous solvents with water.

## Description

### Technical field of the invention

The invention concerns a formulation for the delivery of a hydrophobic active agent in aqueous environment.

### Background of the invention

An active agent is generally understood to be the part of any formulation that produces an intended pharmacological and/or biological effect. In the pharmaceutical field, those agents are typically classified as a function of their solubility in aqueous medium in order to have a first estimation of their bioavailability. Accordingly, an active agent is qualified as being very soluble, freely soluble, soluble, sparingly soluble, slightly soluble, very slightly soluble or practically insoluble, if one part of the active agent can be dissolved in less than 1 part, from 1 to 10 parts, from 10 to 30 parts, from 30 to 100 parts, from 100 to 1000 parts, from 1000 to 10,000 parts or only in more than 10,000 parts of an aqueous medium, respectively. A hydrophobic active agent is generally understood to be an active agent which is sparingly soluble, slightly soluble, very slightly soluble, or practically insoluble.

Poor water solubility has been attributed to almost half of the 150,000 new molecular entities (NMEs) synthesised annually by pharmaceutical companies which is claimed to reduce the performance of more than 10% of successfully marketed drugs (or active agents). Accordingly, several strategies have been developed during these last decades in order to improve solubility of active agents in aqueous medium. The various approaches reported in prior art can be classified in two main categories: a kinetic approach and a thermodynamic approach, the second one being favoured to enhance the long term stability.

In previous work relating to a kinetic approach, hydrophobic active agents have been combined with polymers such as hydroxy propyl methyl cellulose (HPMC), aliphatic polyesters (polylactide, polylactide-co-glycolide, polycaprolactone), gelatin, polyvinyl alcohol, polyvinyl pyrrolidone (PVP), polyethylene oxide, polyvinyl acetate. Those macromolecules, either dissolved in an organic or aqueous phase, can work as stabilizers but can also control the precipitation of hydrophobic active agents by affecting the following physicochemical events: drug nucleation, crystal growth by condensation, or crystal growth by coagulation.

Functional ion-exchange polymers have been also designed to enhance the dissolution rate of hydrophobic active agents by reducing the lattice energy of crystalline form, which can be a critical barrier to promote rapid dissolution in aqueous media. These polymers contain basic or acidic groups that interact with the ionisable molecules of the surrounding medium and exchange their mobile ions of equal charge with surrounding medium reversibly and stoichiometrically. The resultant complex, known as "Resinate", can be formulated as a suspension, dry powder or tablet. A specific feature of these complexes is to be insoluble in aqueous medium, while still being able to release the hydrophobic active agents when exposed to physiological fluids.

The second solubilisation approach relies on a thermodynamic strategy which allows to achieve a true hydrophobic active agent solubilisation, achieving a molecular dispersion in aqueous medium.

A more specific approach consists in the physicochemical association of the hydrophobic active agent with a molecule or an association of molecules to generate what is typically so-called molecular complexes. Macromolecular - drug complexes have been reported with poly(vinyl pyrrolidone), a well-known synthetic polymer which is soluble in water, but also in other various solvents. Thanks to the large dipole moment of its side group, it is promoting dipole-dipole interaction between the polymer and the hydrophobic active agent. Also amphiphilic copolymers made from poly(vinylcaprolactam), poly(vinyl acetate) or poly(ethylene glycol) have been disclosed to form macromolecular soluble complexes and to facilitate dissolution of hydrophobic active agents in aqueous phases.

However, it is well known that until now no universal route has been identified to improve solubility of hydrophobic active agents and no clear relationship can be derived between the molecular properties of hydrophobic active agents and the methods to enhance their solubility. As a result, it has been necessary to adopt a trial and error approach to optimize their formulation.

The main weaknesses of the known formulations developed to enhance the solubility of hydrophobic active agents include:
- their lack of stability with time and after being submitted to physicochemical attacks leading to aggregation and/or drug crystallisation;
- the low hydrophobic active agent loading and concentration;
- the need to add various excipients (such as stabilizing agents or surfactants) typically present in high weight proportion;
- kinetics of release of hydrophobic active agent from a majority of controlled-release formulations present an initial burst instead of a linear rate of release and fail to ensure complete delivery of the hydrophobic active agent;
- local dissolution of the hydrophobic active agent with its excipient but not at the intended site; and
- expensive formulation technologies which are time consuming, induce hydrophobic active agent contamination, are not robust, and which are difficult to scale-up.

A way of ameliorating these problems has been sought.

### Description of the invention

According to this invention there is provided a first embodiment of a complex comprising at least one hydrophobic active agent and an ionic polymer comprising a repetitive unit of formula (I): 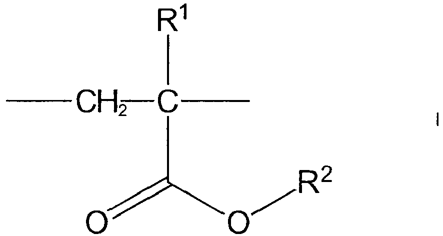
wherein R¹ represents a hydrogen atom or a straight or branched chain alkyl group, preferably a straight or branched chain alkyl group comprising from 1 to 6 carbon atoms, for example a methyl group; R² represents a straight or branched chain alkyl group which is substituted by a group which may have a positive charge at a physiological pH; with the proviso that where the ionic polymer comprising a repetitive unit of formula (I) is poly(2-dimethylamino)ethyl methacrylate having an ionisation percentage of 30% and a molecular weight of 7, 13, 26.4, 49.1, or 91.4 kDa, the hydrophobic active agent is not ovalbumin;
with the proviso that where the ionic polymer comprising a repetitive unit of formula (I) is poly(2-dimethylamino)ethyl methacrylate having a molecular weight of 13 kDa and an ionisation percentage of 30%, 47%, 60%, or 80%, the hydrophobic active agent is not ovalbumin or human insulin;
with the proviso that where the ionic polymer comprising a repetitive unit of formula (I) is poly(2-dimethylamino)ethyl methacrylate having a molecular weight of 10 or 90 kDa, the hydrophobic active agent is not ovalbumin;
with the proviso that where the ionic polymer comprising a repetitive unit of formula (I) is poly(2-dimethylamino)ethyl methacrylate having an ionisation percentage of 30% and a molecular weight of 13, 14, 26.4, 49.1, 90, or 91.4 kDa, the hydrophobic active agent is not human insulin;
with the proviso that where the ionic polymer comprising a repetitive unit of formula (I) is a copolymer of poly(2-dimethylamino)ethyl methacrylate having a molecular weight of 7 kDa and poly(ethylene oxide) having a molecular weight of 0.5 kDa, the hydrophobic active agent is not human insulin;
with the proviso that where the ionic polymer comprising a repetitive unit of formula (I) is poly(2-dimethylamino)ethyl methacrylate having a molecular weight of 10, 20, 40, or 90 kDa and the hydrophobic active agent is human insulin, the complex does not contain sodium dodecyl sulphate;
with the proviso that where the ionic polymer comprising a repetitive unit of formula (I) is poly(2-dimethylamino)ethyl methacrylate having a molecular weight of 10 or 90 kDa and the hydrophobic active agents are P24 structural protein of HIV viral capside and CpG short single-stranded synthetic DNA molecule, the complex does not contain sodium dodecyl sulphate, Tween 20, C8S 04, C11COO-, or PE6800.

According to the invention there is also provided a second embodiment of a complex comprising at least one hydrophobic active agent and an ionic polymer comprising a repetitive unit of formula (I): 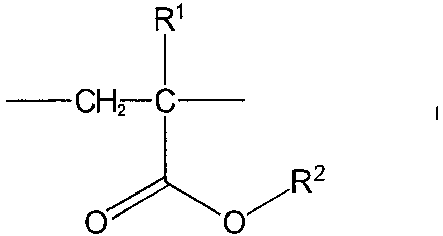 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; wherein the at least one hydrophobic active agent has a molecular weight of from 100 to 1500 g/mol.

According to the invention there is further provided a third embodiment of a complex comprising at least one hydrophobic active agent and an ionic polymer comprising a repetitive unit of formula (I): 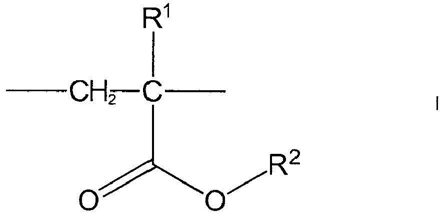 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; wherein the at least one hydrophobic active agent has a molecular weight of from 100 to 1000 g/mol. The active agents listed in the first six provisos of the definition of the first embodiment of a complex according to the invention have a molecular weight which is greater than 1500g/mol.

According to the invention there is further provided a complex for use as a medicament wherein the complex comprises at least one biologically active HA and an ionic polymer comprising a repetitive unit of formula (I) as defined above; or a copolymer thereof; wherein the hydrophobic active agent is not a proteinaceous compound.

According to the invention there is also provided a composition comprising a carrier and a complex comprising at least one HA and an ionic polymer comprising a repetitive unit of formula (I) as defined above; wherein the hydrophobic active agent is not a proteinaceous compound.

According to the invention there is further provided a pharmaceutical composition for use as a medicament, wherein the composition comprises a carrier and a complex according to the invention.

According to the invention there is also provided a method of medical treatment which method includes a step of administering to a human or animal in need of such treatment an effective amount of a complex according to the invention or of a composition according to the invention.

According to the invention there is further provided a method of preparing a complex according to the invention which method comprises the steps of:
(a) dissolving the hydrophobic active agent and the ionic polymer in one or more non-aqueous solvents to form the complex wherein the one or more non-aqueous solvents are miscible with water; and
(b) progressively replacing the one or more non-aqueous solvents with water.

Advantages of the invention include that the ionic polymer enables the formation of a Poly Amphiphilic Complex (PAC) with at least one hydrophobic active agent such that the complex formed has increased solubility compared to the solubility of the at least one hydrophobic active agent alone in aqueous medium. It is also an advantage that the complex formed enhances the resorption of the at least one hydrophobic active agent through biological mucosa and facilitates its crossing of the biological barriers. Advantageously the ionic polymer enables the reversibility of its interaction with the at least one hydrophobic active agent in such a way that as a result of a physiological trigger(s), the hydrophobic active agent can be released under an active form, e.g. at a pre-determined release rate and/or at a pre-determined biological site. In some embodiments, the physiological trigger can be related to a change in temperature and/or a change in local pH or interaction of the complex according to the invention with one or more biological constituents typically known to transport hydrophobic molecules (e.g. plasma proteins or / and lipid structures).

A further advantage is that the ionic polymer used in the invention is characterized by an intermediate solubility such that it is soluble in more than one solvent, including an aqueous medium and one or more of a chlorinated solvent (such as methylene chloride or chloroform), an heterocyclic ether (such as tetrahydrofuran), or dimethylsulfoxide, dimethylacetamide or dimethylformamide. Other types of polymers are known to show an intermediate solubility behaviour, such as poly(ethylene oxide), poly(vinylpyrrolidone), but those macromolecules are not ionic polymers. The solubility of the ionic polymer used in the invention is significantly affected by the charge density, and accordingly by the pH, but also by the nature of the end groups born by the alkyl group R² arising from the ester pending group of each repetitive unit of formula (I).

An additional advantage is that a large dipole moment arising from the side group on the repetitive unit of formula (I) of the ionic polymer used in the invention is well known to promote dipole-dipole interaction between the ionic polymer and a solvent and/or the at least one hydrophobic active agent. Such interactions are therefore contributing to molecular complex formation thanks to their enthalpy contribution. However, according to the state of the art it is also known that dipole-dipole interactions are generally not sufficient to promote complex formation between an ionic polymer and a hydrophobic active agent, in particular if the at least one hydrophobic active agent does not contain polar moieties. The ionic polymer having a repetitive unit of formula (I) can therefore assist to enhance the solubilisation of the at least one hydrophobic active agent thanks to its ability to form a hydrophobic intramolecular cage in which the hydrophobic active agent can be physically entrapped. Without wishing to be bound to a particular theory, it is believed that this local concentration of the hydrophobic active agent inside the complex is entropically driven due to the release of ordered water molecules upon in contact with the hydrophobic active agent, as is known with the solubilisation of drugs within surfactant micelles. Again without wishing to be bound by a particular theory, it is believed that a combination of entropic and enthalpic forces can typically explain the formation of a complex between the polymer and hydrophobic drug, and accordingly enhance the solubility and transportability of plasma proteins for several hydrophobic drugs upon their diffusion within the blood compartment.

A further advantage is that, in contrast to copolymers or multisequence- or multi-segment- based polymers, the multiplicity and the molecular proximity of the potential groups present in the ionic polymer used in the invention offer more interactive binding sites. Accordingly, higher drug loadings may be obtained.

A further advantage is that with the ionic polymer used in the invention, the release rate of the hydrophobic active agent may be pH dependent. This property is advantageous where the complex would encounter a more acidic medium. Due to its specific range of pKa, the ionic polymer used in the invention is able to maintain a buffer capacity of the polymers. Accordingly, in some embodiments, from pH 7 to 8.5 no significant release of the hydrophobic active agent from the complex would be observed. However, if in contact with a more acidic medium, such as the gastric environment in case of oral administration, but also after some cellular internalisation processing known for macromolecules or supramolecular structures or still larger materials (such as liposomes, nanoparticles), the local acidic pH met within endosomic vacuoles can significantly trigger the release of the hydrophobic active agent.

An additional advantage of the invention is that the ionic polymer used in the invention can spontaneously be converted from a cationic polymer, to a polyampholyte polymer, and eventually in a later stage to an anionic polymer. Indeed, if the substituent R¹ (which is chemically linked on the second carbon of the vinyl repetitive unit) is a hydrogen, thus corresponding to an acrylate unit, a self-catalysed hydrolysis of the ester bond occurs spontaneously and progressively when this polymer is in contact with water (Truong, N. P., Jia, Z., Burges, M., McMillan, N. A. J., & Monteiro, M. J. Self-Catalysed Degradation of Linear Cationic Poly(2-dimethylaminoethyl acrylate) in Water, Biomacromolecules, (2011), 12(5), 1876-1882). In this case if originally the corresponding alkyl group R² of the ester is substituted by a ternary or quaternary amino group, the resulting hydrolysis of the ester will generate a carboxylic acid susceptible to be ionized at neutral pH. Accordingly where the ionic polymer comprising a repetitive unit of formula (I) is a copolymer of (a) an ionic polymer comprising a repetitive unit of formula (I) where R¹ represents a hydrogen atom and (b) an ionic polymer comprising a repetitive unit of formula (I) where R¹ represents a straight or branched chain alkyl group, and as a function of a proportion of acrylate repetitive units (a) to repetitive units (b), its global charge could turn progressively from a positive sign to a neutral or even a negative sign. Accordingly, the adjustment of the proportion of acrylate repetitive unit (a) can be used to fine tune the evolution of charge density of the ionic polymer in function of time, allowing therefore to improve the loading of the hydrophobic active agent in function of their own mean charge value, but also its corresponding release rate *in vivo.* This charge inversion of the ionic polymer comprising a repetitive unit of formula (I) could also be valuable in order to reduce a risk of any safety issue. Examples of such safety risks include that cell toxicity could occur in the presence of cationic polymers, especially above a critical molecular weight threshold and if they are present in a biological environment under a free form, i.e. without being associated to anionic or polyanionic molecules, such as peptides, or proteins, or animal cells (Truong, N. P., Jia, Z., Burges, M., McMillan, N. A. J., & Monteiro, M. J. Self-Catalyzed Degradation of Linear Cationic Poly(2-dimethylaminoethyl acrylate) in Water, Biomacromolecules, (2011), 12(5), 1876-1882).

In some embodiments, the complex may comprise at least one hydrophobic active agent and an ionic polymer comprising a repetitive unit of formula (I) as defined above; with the proviso that where the ionic polymer comprising a repetitive unit of formula (I) is poly(2-dimethylamino)ethyl methacrylate, the hydrophobic active agent is not ovalbumin or human insulin; with the proviso that where the ionic polymer comprising a repetitive unit of formula (I) is a copolymer of poly(2-dimethylamino)ethyl methacrylate having a molecular weight of 7 kDa and poly(ethylene oxide) having a molecular weight of 0.5 kDa, the hydrophobic active agent is not human insulin; with the proviso that where the ionic polymer comprising a repetitive unit of formula (I) is poly(2-dimethylamino)ethyl methacrylate and the hydrophobic active agents are human insulin or P24 structural protein of HIV viral capside and CpG short single-stranded synthetic DNA molecule, the complex does not contain a surfactant.

In some embodiments, the definition of the complex according to the invention may be subject to the further proviso that where the ionic polymer is a cationic copolymer based on dimethylaminoethyl methacrylate, butyl methacrylate, and methyl methacrylate with a ratio of 2:1:1, the complex does not comprise an excipient used in an oral dosage form. In some embodiments, the definition of the complex according to the invention may be subject to the further proviso that where the ionic polymer is a cationic copolymer based on dimethylaminoethyl methacrylate, butyl methacrylate, and methyl methacrylate with a ratio of 2:1:1, the complex is not an oral dosage form. The cationic copolymer based on dimethylaminoethyl methacrylate, butyl methacrylate, and methyl methacrylate with a ratio of 2:1:1 is sold under the brand name Eudragit E100, Eudragit E PO, and Eudragit E 12,5 for use as a coating for an oral dosage form which may be a matrix tablet or a multiparticulate. The intention of these provisos is to provide basis for distinguishing that unrelated disclosure.

In some embodiments, where R² is substituted by a ternary amino group which has a mean pKa which is typically close to neutral pH, e.g. from 6.5 to 7.5, e.g. about 6.7. This value being close to physiological pH met in most biological fluids of animal body, the global ionisation level of polymer of formula I is only partial and can be estimated to 30 % at pH 7.4. The nature of the end groups born by the alkyl group R² arising from the ester pending group of each repetitive unit may also affect the pKa of the ternary amino group. These polymers, including homopolymers, are therefore made of at least two different repetitive units, one being positively charged, the other being neutral at pH 7.4, and accordingly can be considered as co-polymers, including when built from a single repetitive unit.

In some embodiments, the ionic polymer of formula (I) may be prepared by living radical polymerization such as atom-transfer radical polymerization (ATRP), to control the macromolecular parameters of the polymer. In some embodiments, the ionic polymer may have a molecular weight which is from 1 kDa, e.g. from 2kDa, to 100 kDa.

In some embodiments, the ionic polymer may have a mean charge density which is the proportion of the number of positively or negatively charged repetitive units to the number of repetitive units. In some embodiments, the mean charge density of the ionic polymer of formula (I) at physiological pH may range from 5 to 100 %, for example a mean charge density of from 10 to 50 %. Several macromolecular architectures can be tailored by living radical polymerisation in order to play on the flexibility of polymer chain, on the separation between segments bearing charges and neutral segments, and on the global hydrophilic/hydrophobic ratio of the polymer.

In some embodiments, the ionic polymer may comprise one single repetitive unit of formula (I). In some embodiments, the ionic polymer may comprise a repetitive unit of formula (I) in combination with one or more other monomers, or other polymers, and/or oligomeric sequences. In some embodiments, the ionic polymer may consist essentially of repetitive units of formula (I) wherein at least some repetitive units of formula (I) bear a charge. In some embodiments, the ionic polymer may comprise (or consist of) repetitive units of formula (I) wherein at least some repetitive units of formula (I) bear a charge. In some embodiments, the ionic polymer may comprise (or consist of) repetitive units of formula (I) wherein from 10% to 70% of the number of repetitive units of formula (I) bear a charge. In some embodiments, the ionic polymer may be an ionic homopolymer which is at least partially charged. In some embodiments, the charge may be a positive charge, in particular a positive charge at physiological pH. In some embodiments, the ionic polymer may be a cationic homopolymer. In some embodiments, the complex may have a dynamic light scattering size of from 1, e.g. from 10, to 5000, e.g. to 4000, e.g. to 3000, e.g. to 2000, e.g. to 1000, e.g. to 500, e.g. to 300, e.g. to 200 nm. In some embodiments, the complex may have an aqueous concentration of from 1mg/ml, e.g. from 5mg/ml, e.g. from 10mg/ml to 200mg/ml, e.g. to 150mg/ml, e.g. to 100mg/ml, e.g. to 50mg/ml.

In some embodiments, the ionic polymer may be an ionic polymer of formula: 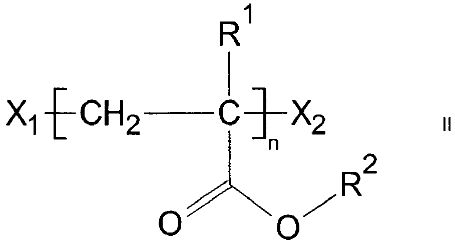 wherein X₁ and X₂ each respectively represent the alpha and omega end groups of the polymer; R¹ and R² are each as defined for the repetitive unit of formula (I); and n represents the number of repetitive units of the ionic polymer.

In some embodiments, the ionic polymer may have a mean pKa of from 6 to 8, for example a pKa which is around physiological pH which is about 7.4. The relatively low density of ionic groups present on the polymer at physiological pH is advantageous to reduce risk of any safety issues. Indeed, an advantage of this embodiment is that it ameliorates a problem with PEI which is that branched PEI has a pKa value of from 8.2 to 9.9 which in combination with its molecular weight (Mw) and branching level, results in a too high charge density of positive groups which is believed to cause cytotoxicity.

In some embodiments, the ionic polymer may have alpha and omega groups respectively represented by X₁ and X₂. It is well known by a person of skill in the art that the alpha and omega groups represented by X₁ and X₂ are affected by the nature of the reaction conditions used for synthesis of the ionic polymer such as the initiator, nature of co-monomers, if present, and conditions of polymerisation termination. In some embodiments, the ionic polymer may be formed by radical polymerisation, and in particular by atom-transfer radical polymerization (ATP) in which these factors are well known according to the state of the art. In some embodiments, where a co-monomer is not present, X₁ may represent an ethyl isobutyrate moiety and X₂ may represent a hydroxyl group.

In some embodiments, R² represents a straight or branched chain alkyl group comprising from 1 to 10 carbon atoms, for example from 1 to 6 carbon atoms, e.g. two carbon atoms. In some embodiments, the optional group for R² which has a positive charge at physiological pH is a group of formula: -N(R³)₂ (III) wherein each R³ substituent may be the same or different and represents a hydrogen atom or a straight or branched chain alkyl group. In some embodiments, each R³ substituent may represent a straight or branched chain alkyl group to increase hydrophobic active agent loading and/or release kinetics profile, but also pH dependence. In some embodiments, the straight or branched chain alkyl group represented by R³ may have from 1 to 6 carbon atoms, for example from 1 to 4 carbon atoms; particularly R³ may represent a methyl group.

In some embodiments, the repetitive unit of formula (I) may be positively charged where the R² substituent represents a straight or branched chain alkyl group which is substituted by a group which has a positive charge, for example a protonated group of formula (III) or a group of formula -NH(R³)₂⁺ (IV). The protonated group of formula (III) may be formed when the ionic polymer is dispersed or dissolved in a physiologically compatible medium such as an isotonic saline solution or phosphate buffer saline medium (PBS). The group of formula (IV) may be formed during the preparation of the polymer, for example during a purification step in a basic solution. In some embodiments, the mean charge density may be from 1, for example from 20, e.g. from 15 to 80 %, for example to 50 %, e.g. to 30%.

In some embodiments, where the ionic polymer is positively charged, the ionic polymer may include a counter ion which is an anion, for example OH⁻; Cl⁻; HCO₃⁻; NO₃⁻; and/or H₂PO₄⁻.

By co-polymer is meant a polymer comprising two or more repetitiveunits where the repetitive units can be organized according to a different architecture (for example linear, cyclic, grafted and/or star), sequence (for example random, block copolymer and/or micro- sequence), and/or configuration (for example atactic, isotactic and/or syndiotactic). In some embodiments, the composition of the ionic polymer may be altered by copolymerisation of monomers bearing positive charges but also by non-ionic or/and anionic groups.

In some embodiments, the ionic polymer may be a copolymer in order to modify the charge density of the ionic polymer and to limit protein interaction. In some embodiments, the repetitive unit of formula (I) may be copolymerised with one or more of the following additional repetitive units: an ethylene glycol, an acrylate, a methacrylate, optionally carrying a polyethylene oxide (PEO), for example with a mean Mw typically from 400 to 5,000, or a repetitive unit of formula (I) wherein R² is substituted by a group of formula (IV). In some embodiments, the co-monomer may be randomly distributed or the co-polymer may have a block-wise or multi-block structure. In some embodiments, the proportion and distribution of the additional repetitive units should be adjusted in order that the iconic copolymer is soluble in an aqueous medium. In some embodiments, the nature and distribution of the additional repetitive units may be selected to adjust the hydrophilic to lipophilic balance (HLB) of the copolymer formed to finely tune its affinity to a wide variety of hydrophobic active agents which could differ in chemical architecture, mean composition, chemical functionalities, molecular weight and solubility in aqueous medium and organic medium. In view to quantify this last property, the LogP is typically used to quantify the hydrophobicity of a given molecule. LogP represents the partition coefficient of a molecule between an aqueous and a lipophilic phase, usually octanol and water. This parameter can be determined experimentally by measuring the concentration of a given active in the two liquid phase after achieving its equilibration. Alternatively, it can also be calculated. In some embodiments, the hydrophobic active agent may be sparingly soluble, slightly soluble, very slightly soluble or practically insoluble. In some embodiments, the hydrophobic active agent may have a LogP value which is from 2 to 8.

Examples of copolymeric and terpolymeric structures which could be used as the ionic polymer include poly[2-(dimethylamino)ethyl methacrylate)-co-acrylic acid] **(III),** poly[2-(dimethylamino)ethyl methacrylate)-co-methacrylic acid] **(IV),** poly[2-(dimethylamino) ethyl methacrylate)-co- poly(ethylene glycol) a- methyl ether, co-acrylate] **(V),** poly[2-(dimethylamino) ethyl methacrylate)-co-poly (ethylene glycol) a- methyl ether, co-methacrylate] **(VI),** poly[2-(dimethylamino) ethyl methacrylate)-co-poly(ethylene glycol)] **(VII),** poly[2-(dimethylamino) ethyl methacrylate)-co- poly(ethylene glycol)] **(VIII),** poly[methacrylic acid-co-2-(dimethylamino) ethyl methacrylate)-co- poly(ethylene glycol) α-methyl ether, co-methacrylate] **(IX),** poly[methyl methacrylate-co-2-(dimethylamino) ethyl methacrylate)-co-poly(ethylene glycol) α-methyl ether, ω-methacrylate] (X), poly[trimethylamino) ethyl methacrylate -co-2-(dimethylamino) ethyl methacrylate)-co- poly(ethylene glycol) α-methyl ether, co-methacrylate] **(XI),** poly[trimethylamino) ethyl methacrylate -co-2-(dimethylamino) ethyl methacrylate] **(XII),** poly[2-(dimethylamino) ethyl methacrylate-co-methyl methacrylate-co-butyl methacrylate] of formula **(XIII)** wherein the molar ratio of methyl methacrylate to the copolymer is at most 10mol% (as defined by m) and the molar ratio of butyl methacrylate to the copolymer is at most 10mol% (as defined by 0); and poly[2-(trimethylamine) ethyl methacrylate chloride-co-ethyl acrylate-co-methyl methacrylate] of formula (XIV) wherein the molar ratio of ethyl methacrylate to the copolymer is at most 10mol% (as defined by m) and the molar ratio of methyl methacrylate to the copolymer is at most 10mol% (as defined by 0).

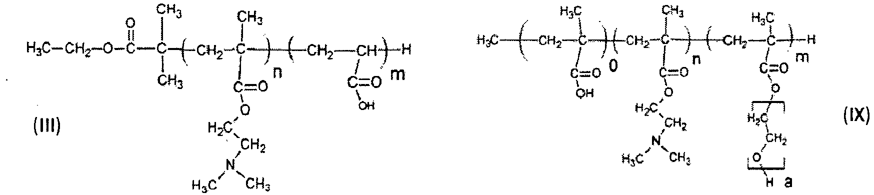

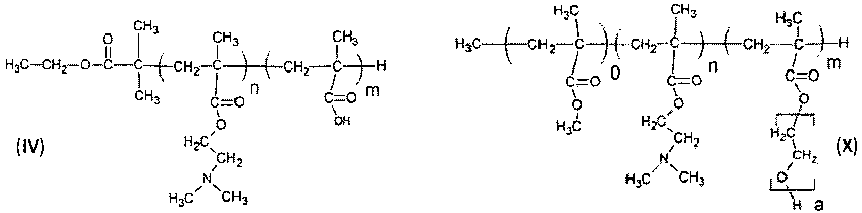

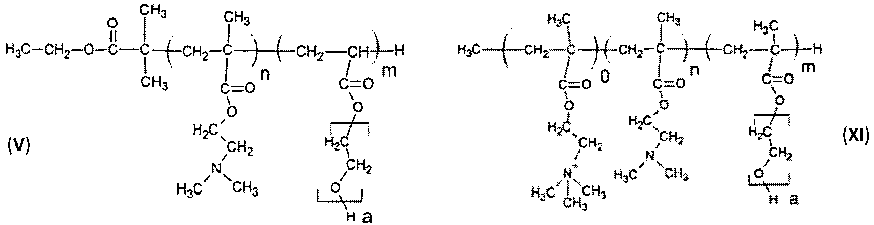

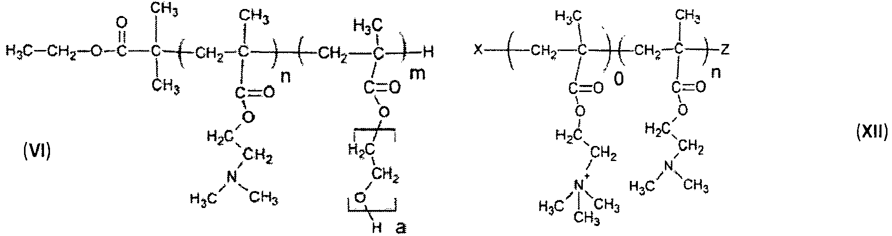

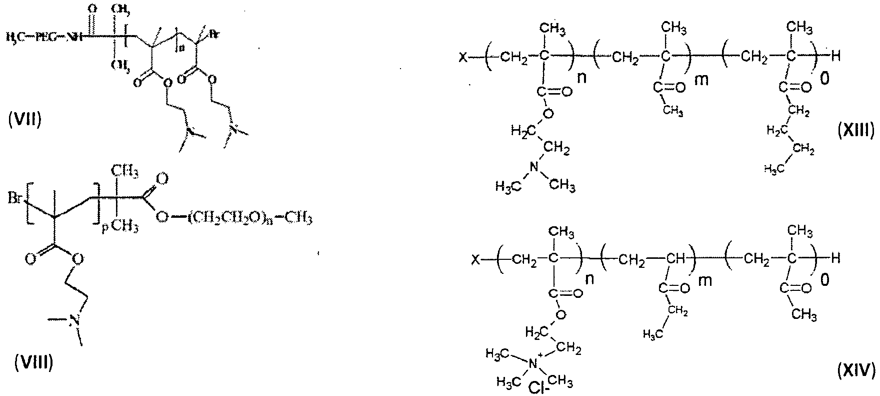

In some embodiments, the ionic polymer may be a haemocompatible polymer. By haemocompatible, it is meant that the ionic polymer does not elicit a substantial adverse reaction when in contact with a patient's blood or blood compartment. The criteria for haemocompatibility are detailed in ISO 10993-4 and include, for example, that there is substantially no haemolysis, no activation or inhibition of coagulation cascade, no complement activation and/or no cell activation aggregation (specifically for platelet).

Unless otherwise defined, the molecular weight (Mw or Mn) or molecular mass is expressed in Daltons (Da). In some embodiments, the ionic polymer may have a linear structure, particularly of the family of poly(meth)acrylates. In some embodiments, n represents an integer which may have a value of from 6 to 130 such that the ionic polymer of formula (II) may have a molecular weight of from 1,000, for example from 2,000, e.g. from 4,000 to 20,000, for example to 15,000, e.g. from 4,000 to 20,000, for example to 100,000.

The ionic polymer offers several advantages over naturally occurring polycations. In particular due to their synthetic origin, their macromolecular features can be adapted in a versatile way. For example, their mean charge density and charge distribution can be easily modified by using at least substituted and unsubstituted repetitive units whose molar proportion and distribution within the polymer of formula (II) may be random or can be tailored to control the interaction of the ionic polymer with a defined active agent.

In some embodiments, the repetitive unit of formula (I) may comprise N,N dimethyl amino ethyl methacrylate (such that the ionic polymer of formula (II) is PolyDimethyl Amino Ethyl MethAcrylate or PDMAEMA). In some embodiments, PDMAEMA may have a mean charge density of about 30 %.

With an exponential factor of the Mark-Houwink parameters of 0.5 and 0.6, linear PDMAEMA has a relatively high expanded conformation, a macromolecular feature allowing the interaction with an active agent. With a mean pKa of around physiological pH which is about 7.4, PDMAEMA has a relatively low mean charge density compared to PEI or poly(L)Lysine. Accordingly, its haemocompatibility and cytotoxicity is superior compared to polycations bearing too many positive charges on their backbone.

As additional advantages, the ionic polymer is easy to produce at an industrial scale and at a very low cost. Its macromolecular characteristics and purity can be easily controlled by macromolecular engineering which allows the adjustment of their composition, length, and sequence.

In some embodiments, the complex may have a surface modification. Advantages of providing a surface modification include that their clearance is reduced and their cellular uptake is promoted. In some embodiments, the surface modification may be with an anionic hydrosoluble molecule, for example heparin. In some embodiments, the concentration of the surface modification in the complex may be from 0.001wt/vol%, e.g. from 0.01wt/vol%, to 1wt/vol%, e.g. to 0.1wt/vol%.

In some embodiments, the complex or composition according to the invention may be in lyophilisate or spray-dried form. A skilled person would know how to prepare a suitable lyophilisate or spray-dried form of the complex or composition according to the invention. The lyophilisate or spray-dried form of the complex or composition according to the invention could be reconstituted in a buffered isotonic medium just before administration.

In some embodiments, the composition according to the invention may be an isotonic solution, for example a sterile isotonic solution which is optionally buffered at pH 7.4. In some embodiments, the pharmaceutically acceptable carrier may be an optionally buffered isotonic medium, for example a phosphate buffered saline medium (PBS). A suitable PBS composition is for example: composition: KH₂PO₄ 1.4 mM, Na₂HPO₄ 10 mM, NaCl 137 mM, KCI 2.7 mM and adjusted to pH 7.4. A skilled person would know how to prepare a suitable optionally buffered, sterile isotonic solution of the complex or composition according to the invention.

The composition may have a concentration of the complex of from 1 µg/mL and 100 mg/mL. The concentration of the complex or of the at least one active agent in the composition according to the invention may be from 10 to 1000 times higher than the final concentration requested to be achieved for its final application.

In some embodiments, the complex or composition may comprise one or more hydrophobic active agents. In some embodiments, the hydrophobic active agent may be an organic compound with a molecular weight below 1,500 Da. In some embodiments, many different hydrophobic active agents can be incorporated within the formulation. In some embodiments, since the PAC is designed for delivery of actives for very wide applications, the at least one active agent may be a compound having an activity on the human or animal body. Examples of suitable hydrophobic active agents include an immunomodulator, cytokine, hormone (for example: antitumor agent, such as doxorubicin), a clotting factor, and/or a low molecular weight hydrophobic peptide (e.g. a hydrophobic peptide having a molecular weight of less than 1000 g/mol). Examples of hydrophobic active agents having an active effect on the human or animal body for use in the present invention include a hydrophobic active agent for treatment of osteoporosis, bone metabolism improver, hypnotic sedative, sleep inducing agent, anxiolytic, antiepileptic, antidepressant, anti-Parkinson agent, agent for neuropsychiatry, central nervous system agent, local anaesthetic, skeletal muscle relaxant, autonomic agent, antipyretic analgesic, anti-inflammatory agent, antispasmodic agent, vertiginous agent, cardiotonic, antiarrhythmic agent, diuretic, antihypertensive agent, vasoconstrictor, vasodilation, agent for circulatory organs, agent for hyperlipidemia, respiratory stimulant, antitussive, antitussive expectorant, bronchodilator, intestinal agent, peptic ulcer agent, stomach digestive agent, antacid, laxative, gastrointestinal agent, adrenal hormone preparation, hormonal agent, vitamin, hemostatic agent, liver disease agent, diabetic agent, antihistamine agent, antibiotic, antibacterial agent, anti-mosquito agent , anthelminthic agent, antimalarial agent, and/or antineoplastic agent. Names of suitable hydrophobic active agents include: ticagrelor, ivermectin^{®}, curcumin^{®}, 5-aminoosalicylic acid, acaclovir, adinazolam, ascorbic acid, aspirin^{®}, acetylsalicylic acid, acasol Minophene, Acetobutol, Acetohexamide, Atenvorrost, Atorvastatin^{®}, Apomorphine, Aminopyrine, Aminophylline^{®}, Ethylaminobenzoate, Amrinone, Amobarbium Oxide, Albuterol^{®}, Alprazolam^{®}, Valopurinol, Ampicillin^{®}, Ambroxol^{®}, Etenzamido, ethosuximide, Etomidorin, ephedrine^{®}, erythromycin^{®}, O alkoxy tetracycline, Kosifurazone, omeprazole^{®}, carmofur, quinidine^{®}, quinine^{®}, griseofulvin^{®}, glipizide^{®}, glucagon^{®}, dalbenclamide, chloramphenicol^{®}, chlordiazepoxide, chlorothiazide^{®}, ketoconazole^{®}, colestimide, codin^{®}, cobamamide, colchicine^{®}, zafirukast, Diazepam^{®}, diquitoxin, diclofenac^{®}, diclofenac sodium^{®}, cyclophosphamide^{®}, digoxin^{®}, cicotiamine, dipyridamole, cimetidine^{®}, josamycin^{®}, simvadin^{®}, scalfarat, scopolamine^{®}, spironolactone^{®}, sulpiride^{®}, sulfasalazine, sulfadimethoxine, sulfamethizone Sulfaguanidine, sulfamethoxazole, sulfur Soxazol, cefotetan, cefuroxime^{®}, Itraconazole^{®}, selegiline^{®}, celecoxib^{®}, tasosartan, tiotepa^{®}, theophylline^{®}, dextromethorphan^{®}, tetracycline^{®}, teprenone, terfenadine, terbium phosphorus, doxorubicin^{®}, tramadol etidrol, triamcinolone^{®}, triamteren, Fenofibrate^{®}, tlutamide, nateglinide, Naproxen^{®}, nicotinic acid amide, nitroglycerin^{®}, nitrofurantoin^{®}, diphadipine, nomonapride, noscapine, hydocorticoid, pafenorol, valdecoxib, valproate^{®}, haloperidol^{®}, hydrocortithine, hydrocortisone^{®}, atovaquone^{®}, Faropenem sodium, Famotidine^{®}, Phenacetin, Phenytoin^{®}, Pheny Pancreas, Primodone, Fluorouracil^{®}, Methylprednisolone^{®}, Prednisolone^{®}, Prednisone^{®}, Phytohormone^{®}, Phenidobarbital, Fenoprofen Calcium, Telmisartan^{®}, Pseudoephedrine^{®}, Budesonide^{®}, Phoformoterol Fumarate, Prodynesemide, probenecid^{®}, bromwareril urea, betamethasone^{®}, penicillin, peroxetine, perfenadine, benzyl penicillin^{®}, penus isosin, calcium hospatenate, polythiazide, chlorophyllumine maleate, midazolam^{®}, milnacipran^{®}, doxazosin mesylate, methyldopa^{®}, metoclobulamide, methotrexate^{®}, metoprolol^{®}, mepirisol Morphine, Ciprofloxacine^{®}, ranitidine^{®}, lansoprazole^{®}, ricinovir, risperidone^{®}, lisoflavin, lidocaine^{®}, codin phosphate, dimorphorphan phosphate, pyridoxal phosphate, reponorussone monel, reserpine^{®}, repodopa, orobaspin, lorazepam^{®}, orobaspin, warfarin^{®}, aclarubicin hydrochloride, Amitriptyline hydrochloride, amosulalol hydrochloride, ampicillin phthalidyl hydrochloride, indenoloyl hydrochloride, ethamptol hydrochloride, indansetron hydrochloride, daranisetron hydrochloride, dulanisetron hydrochloride, chlorpromazine hydrochloride, diphenhydramine hydrochloride, dibecephine hydrochloride, dibein hydrochloride, hydrochloric acid evening mouth, tiapride hydrochloride, terazosin hydrochloride , Bicardipine hydrochloride, barnidipine hydrochloride, hydralazine hydrochloride, bifuemerane hydrochloride, prazosin hydrochloride, hydrochloric acid Pafenon, hydrochloric Moperone, ranitidine hydrochloride, hydrochloric Ramose Tron, scopolamine butylbromide, isosorbide nitrate^{®}, quinidine nitrate, thiamine mononitrate, and/or chloral hydrate (where "^{®}" indicates a registered trademark).

In some embodiments, the at least one hydrophobic active agent may be a synthetic hydrophobic active agent (e.g. produced according to a chemical route) or a hydrophobic active agent (e.g. a biological compound) from a natural source.

Further advantages of the invention include that the complex and composition may be prepared in a straightforward way without requiring any expensive mechanical tools and in some cases without needing any hazardous organic solvents. In some embodiments, the preparation of the complex and the composition generally comprises physical mixing of the ionic polymer with at least one hydrophobic active agent (for example, an aromatic or heterocyclic organic molecule or a hydrophobic peptide drug).

Where a hydrophobic active agent has an aqueous solubility which is at least 10 µg/mL, its molecular association with the ionic polymer used in the invention would still be feasible directly in an aqueous medium, without needing additional formulation procedures. In this case, the addition of an aqueous phase to the polymer powders and to the hydrophobic active agent under a solid form could be enough in order to solubilize them under the form of soluble and stable PAC. Accordingly the original equilibrium existing free solid drug in suspension and free drug in solution will be progressively shifted towards the formation of hydrosoluble drug - polymer complexes.

Where the aqueous solubility properties of the hydrophobic active agent is too low, i.e. typically below 10 µg/mL, it should be understood by someone knowing the state of the art, that the molecular complexation between this category of hydrophobic active agent and the ionic polymer will be too slow kinetically if performed in an pure aqueous medium. In the latter case, a two step-procedure according to the invention could be used where the PAC will be made first in a non-aqueous solvent, or in a binary non-aqueous solvent system, whose nature will be selected to promote dissolution and molecular interaction between the hydrophobic active agent and the polymer. These two solvents should be miscible. Moreover, they must also be miscible with water. Examples of solvents to be used in the binary mixture include: THF, DMSO, DMF, isopropyl alcohol, acetone, ethanol, methanol, propylene glycol, ethylene glycol or polyethylene glycol (e.g. having a Mw of 350). Their selection should take into consideration the LogP of the hydrophobic active agent and the solubility of the ionic polymer in order to promote their rapid dissolution in the binary solvent and their interaction. Additionally, their selection should be appropriate in order to avoid a possible efflux of free hydrophobic active agent during purification described just afterwards. In a second step, the solvent(s) progressively eliminated without impairing the final aqueous solubility of the PACdue to the hydrophilic domains exhibited by the polymer. Elimination of the solvent(s) can be realized according to any purification techniques which are compatible with mixtures of aqueous or organic mixture and which allows easy elimination of the solvent(s) and free drug from the PAC. Examples of suitable purification techniques include dialysis or ultrafiltration can be mentioned to be used to purify PAC at a laboratory- or industrial- scale, respectively. For example, progressive elimination of the solvent is performed by dialysis or ultra-filtration during 1 to 24 hours, for example 15 hours. In some embodiments, progressive elimination means from 1 hour till 24 hours, for example from 5 hours to 15 hours. Advantages of the two step procedure according to the invention include that the size of the complex according to the invention may be reduced.

In some embodiments, the complex according to the invention can be dried after purification in order to improve the stability of the formulation and to facilitate their storage and transport. For this purpose, the optionally purified PAC aqueous formulations can be freeze-dried or spray-dried according to standard protocols suitable for various industries.

In some embodiments, a dried and optionally purified complex according to the invention may have a Dynamic Light Scattering (DLS) size range of from about 1 nm, e.g. from 1 nm to about 10 nm in cases of formation of totally hydrosoluble PAC (e.g. PACs having an aqueous solubility which is at least 10 µg/mL). In some embodiments, a dried and optionally purified complex according to the invention may have a Dynamic Light Scattering (DLS) size range of from 10 nm to 5000 nm in cases of insoluble PACs (or nanoaggregates of PACs) having an aqueous solubility which is less than 10 µg/mL. In some embodiments, a dried and optionally purified complex according to the invention may have a Dynamic Light Scattering (DLS) size range from 50, e.g. from 100, e.g. from 200 to 5000 nm in the case of formation of PACs which are made from a mixture of hydrosoluble PACs and nanoaggregates of PACs or only made from nanoaggregates of PACs.

In some embodiments, the complex according to the invention may be sterilised. If the complex comprises a hydrophobic active agent which has a solubility of at least 10 µg/mL (such that they are totally hydrosoluble) with a mean size of 10 nm or below, or are composed of nanoaggregates with a DLS size below 200 nm, they can be easily sterilized by sterile filtration, either before drying or just before application. Alternatively, if the mean size of the PAC is above 200 nm, their sterilisation can be performed by other sterilisation techniques which are well-known from the medical and pharmaceutical industries, e.g. ethylene oxide treatment, beta or gamma sterilisation or heat sterilisation.

### Applications

The complex (or PAC) and/or composition according to the invention can be administered through different ways including but not limited to oral, nasal, pulmonary, topical (or skin), or parenteral administration in order to enhance the diffusion of drug through mucosal barrier. Moreover, where the complex and/or composition according to the invention have a minute size and are haemocompatible, they can be diluted in an isotonic physiological solution also customized in order to be injectable parenterally, subcutaneously, intramuscularly, or intravenously in view to facilitate their access to their pharmacological sites, including for the purpose to cross the Blood-Brain-Barrier (BBB). More specifically the administration of the complex and/or composition according to the invention nasally may promote their resorption in the systemic circulation and can also give direct access to the central nervous system (CNS), therefore avoiding the difficult passage of the BBB. The haemocompatibility and cell biocompatibility of the ionic polymer has been previously reported in the literature.

In some embodiments, depending on the mode of administration, the composition according to the invention and/or the complex (or PAC) according to the invention may be delivered in the form of: a suspension (for example in an isotonic medium buffered at neutral pH), an aerosol (e.g. for nasal, pulmonary, or topical/skin application), a viscous liquid (e.g. for intramuscular or other parenteral routes in order to limit their diffusion and increase their local therapeutic action at the site of injection), a liquid able to generate a gel once injected within the body, or a gastro-resistant capsule. In some embodiments, a gastro-resistant capsule may have a pH-sensitive polymer coating which is selected to dissolve after passage in the small intestine to release the complex. In some embodiments, the complex (or PAC) and/or composition according to the invention may be immobilized in a gastro-resistant formulation to protect the complex from a gastric environment.

The invention will now be illustrated with reference to the following Figures of the accompanying drawings which are not intended to limit the scope of the claimed invention:

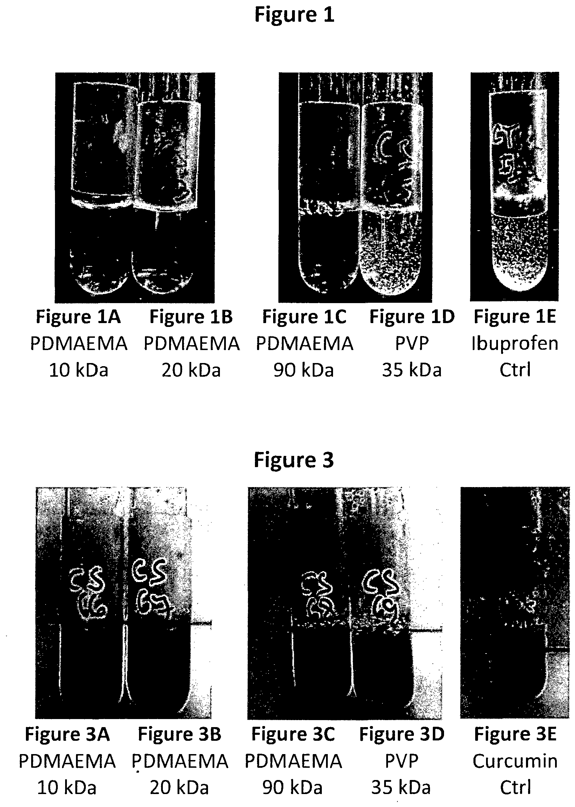

**Figures 1A to 1E** illustrate the dissolution state of ibuprofen (5 mg/mL) when mixed with ionic polymer made from PDMAEMA with Mw of 10, 20 or 90 kDa (45 mg/mL) according to example 1 (Figures 1A, 1B, and 1C respectively). In contrast either with PVP K30 (BASF) (45 mg/mL) (Figure 1D) or without any excipient (Figure 1E), ibuprofen does not dissolve at all remaining under the form of macroscopic crystals in suspension in water;

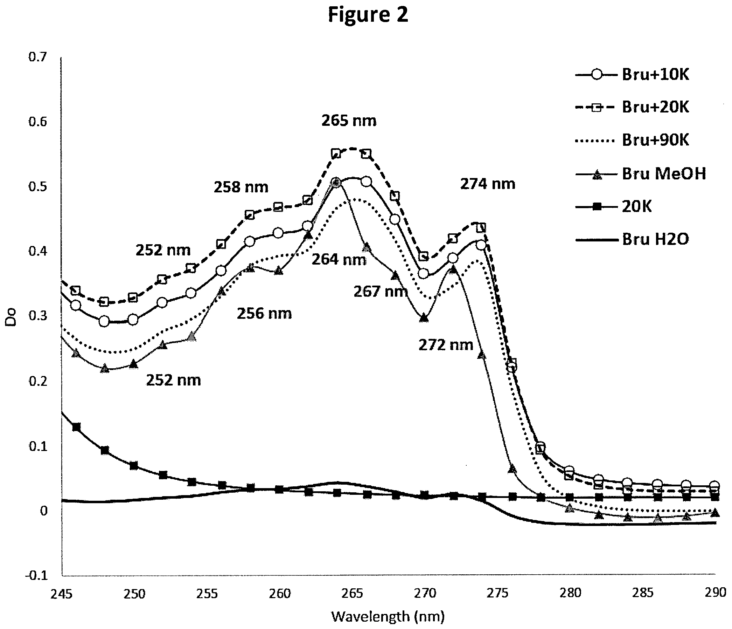

**Figure 2** illustrates the UV spectra (245 to 290 nm) of PAC loaded with ibuprofen after its dissolution in water according to example 1. Three UV spectra are given as controls: i) free ibuprofen dissolved in methanol ii) free ibuprofen dissolved in water iii) PDMAEMA dissolved in water;
**Figures 3A to 3E** illustrate the dissolution state of curcumin (5 mg/mL) when mixed with ionic polymers made from PDMAEMA with Mw of 10, 20 or 90 kDa (45 mg/mL) according to example 3 (Figures 3A, 3B, and 3C respectively). In contrast, either with PVP K30 (BASF) (45 mg/mL) (Figure 3D) or without any excipient (Figure 3E), curcumin dispersions do not disclose any colour change and give rise to rapid sedimentation of the curcumin agglomerates;

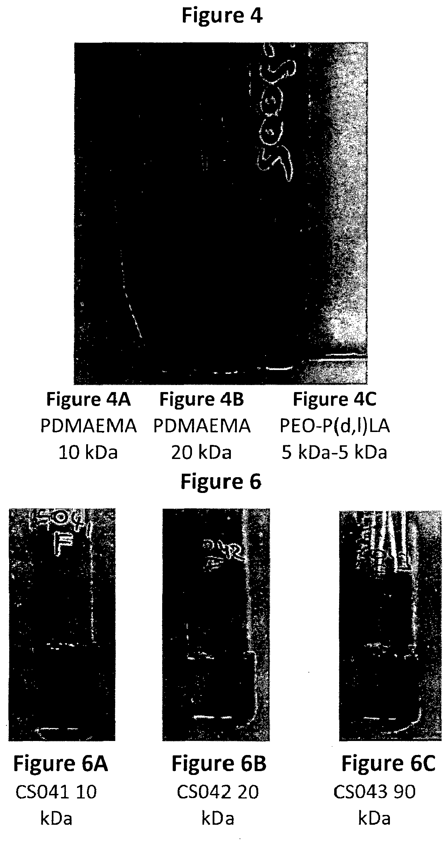

**Figure 4** illustrates the dissolution state of curcumin (5 mg/mL) when mixed with ionic polymers made from PDMAEMA with Mw of 10 or 20 kDa (45 mg/mL) (Figures 4A and 4B) according to example 4. In contrast, a formulation control made adopting the same procedure but using a di-block copolymer made of a PEO-P(d,l)LA) di-block copolymer instead of an ionic polymer does not disclose any colour change (Figure 4C);

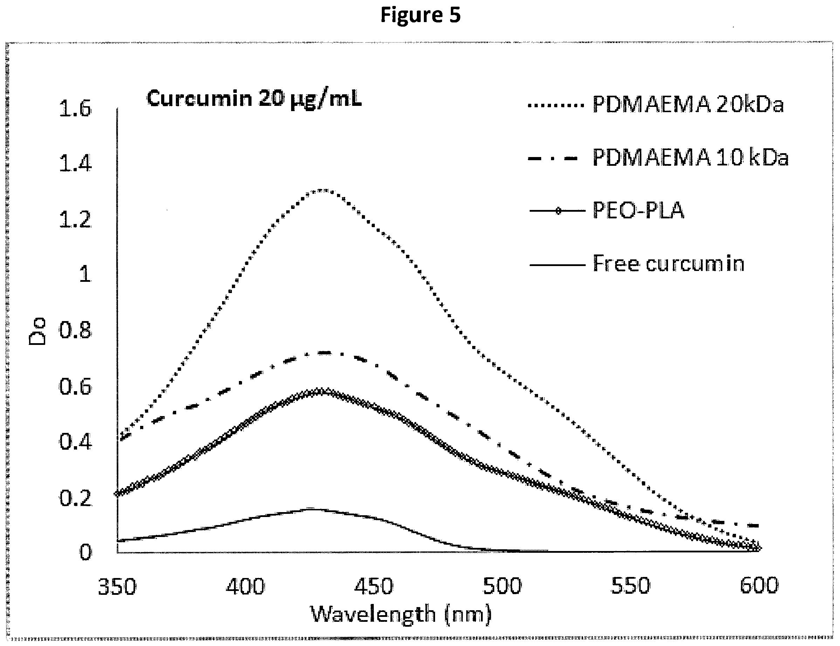

**Figure 5** illustrates the visible absorbance spectra (350 to 600 nm) of two PACs loaded with curcumin (10 %) prepared according to example 4 and after their dilution in water. Two UV spectra are given as control: i) free curcumin dissolved in DMSO (1 mg/mL) and diluted afterwards in water; and ii) curcumin formulation according to example 3 but using a di-block copolymer made of a PEO-P(d,l)LA) di-block copolymer instead of an ionic polymer;
**Figure 6** illustrates the dissolution state of atovaquone (5 mg/mL) when mixed with ionic polymers made from PDMAEMA with Mw of 10, 20 or 90 kDa (45 mg/mL) according to example 7 (Figures 6A, 6B, and 6C);

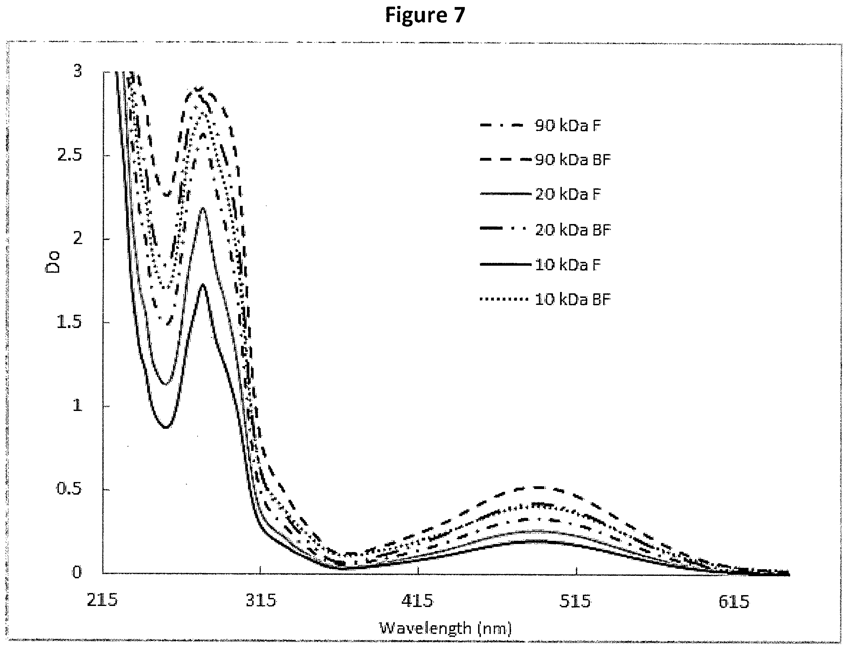

**Figure 7** illustrates the UV - visible spectra (245 to 290 nm) of PACs made from PDMAEMA with Mw of 10, 20 or 90 kDa and loaded with atovaquone (10 %) after freeze-drying and redissolution in water according to example 7. The UV spectra have been taken on the PAC solutions before (BF) and after (F) are filtration on 0.2 µm polysulfone filter;

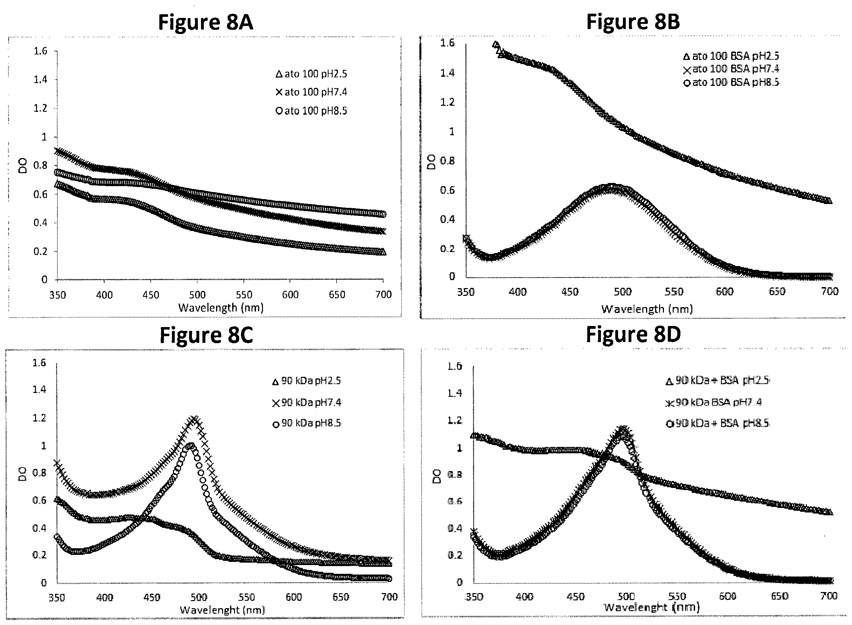

**Figures 8A to 8D** illustrates the evolution of the visible absorption spectra (350 to 700 nm) of atovaquone either under a free form (after dissolution in DMSO and diluted 10 times in PBS **(****Fig 8A****)** or in BSA solution (10 mg/mL) **(****Fig 8B****),** either after PAC formulation according to example 8 with an ionic polymer (90 kDa) and diluted in PBS to achieve a concentration of atovaquone of 100 µg/mL in water **(****Fig 8C****)** or in BSA solution **(****Fig 8D****).** The pH of the PBS has been adjusted to 2.5, 7.4 or 8.5 and the visible spectra have been taken 1 h after dilution in PBS;

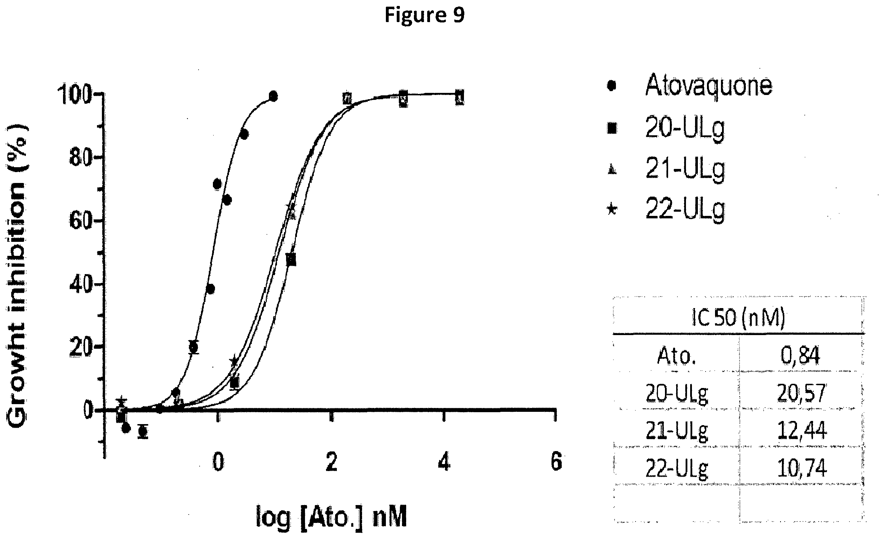

**Figure 9** illustrates the results of the in vitro pharmacological activity of atovaquone (free form (labelled Atovaquone) or loaded within PAC made of PDMAEMA differing in Mw: 10, 20 or 90 kDa (labelled 20-ULg;
21-ULg; and 22-ULg respectively). This growth inhibition assay was carried out on 3D7 cells synchronized at ring stage and the results are expressed in terms of drug concentration requested to inhibit 50 % of the cell population (IC50) according to Example 9; and

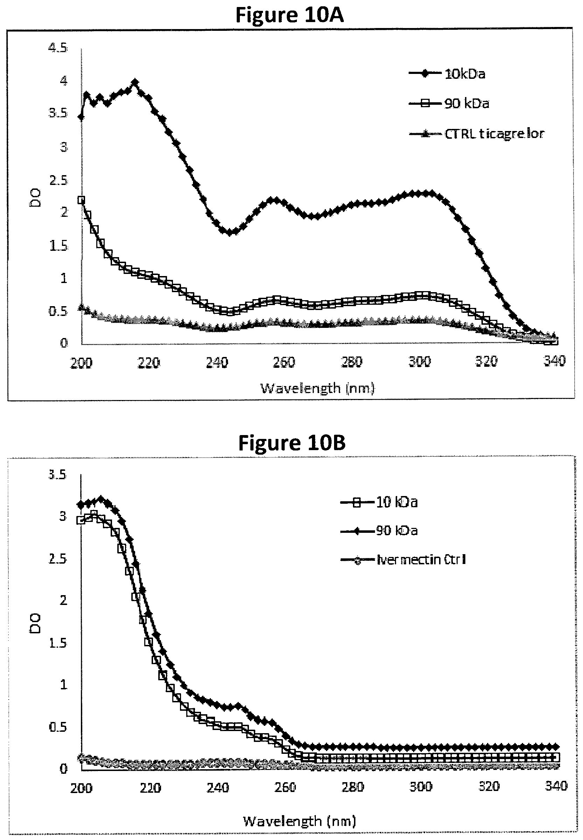

**Figure 10** illustrates the UV spectra (200 to 340 nm) of PACs made from PDMAEMA with Mw of 10 or 90 kDa and loaded with either ticagrelor (Fig 10A) or ivermectin (10 %) (Fig 10B) after freeze-drying and redissolution in water according to example 11. UV spectra have been taken also on control formulations (indicated by the abbreviation "Ctrl") made using the free drugs but without ionic polymer and using the same formulation process disclosed in example 11.

The invention is illustrated with reference to the following Examples which are not intended to limit the scope of the claims.

### EXAMPLES

Our formulations were tested with several hydrophobic actives, namely ibuprofen, curcumin, atovaquone, ivermectin, ticagrelor and 7 hydrophobic peptides. The formation and stability of these PACs was analysed with the following methods:
(a) Dynamic Light Scattering (DLS) giving the mean light scattering Intensity (Id) and autocorrelation function evolution, indicates the formation of the complex and its size as well as stability of PAEC (ionic strength, time, mechanical stress);
(b) ¹H.NMR and UV spectroscopy to analyse active loading;
(c) In vitro release kinetics of the actives from PAC;
(d) Visible spectrometry to analyse the interaction of proteins with PAC;
(e) In vitro assays to analyse the bioavailability and pharmacological action of PAC; and
(f) In vivo assays to analyse the bioavailability and pharmacological action of PAC.

### EXAMPLE 1: Preparation of Poly Amphiphilic Complexes loaded with ibuprofen by direct dissolution in an aqueous phase

Ibuprofen is a hydrophobic drug considered as practically insoluble in water with a solubility of 0.21µg/mL at 25°C in water and a LogP equal to 3.97. Ibuprofen is the most commonly used and most frequently prescribed Non-Steroidal Anti-Inflammatory Drug (NSAID). It is a non-selective inhibitor of cyclo-oxygenase-1 and cyclo-oxygenase-2 and it has a prominent analgesic and antipyretic role on human.

PACs are prepared by physical mixing of an aqueous medium, for example water or a sodium phosphate buffer medium equilibrated at a pH of 7.4 at room temperature with ibuprofen and an ionic polymer made of poly(2-dimethylamino)ethyl methacrylate (PDMAEMA), with a molecular weight of 10 kDa, 20 kDa or 90 kDa. The final concentration of ibuprofen and of PDMAEMA has been fixed to 5 mg/mL and 45 mg/mL, respectively, to achieve a 10 % drug loading. These two compounds are first weighed in given receptacle, for example a Pyrex tube of 5 mL before making a single addition of the pre-determined volume of aqueous phase to achieve the intended concentrations. To promote dissolution, the Pyrex tube is agitated for 15 s under a vortex agitator before applying a rotational agitation for at least 2 h at room temperature. This procedure can be applied in an extended active concentration ranging at least between 0.1 to at least 10 mg/mL, depending on the final drug loading, typically 10 to 50 wt %.

For the sake of comparison, a sample was prepared adding ibuprofen crystal alone in water, thus without PDMAEMA and a sample was prepared with polyvinyl pyrrolidone (PVP K30 from BASF) instead of PDMAEMA.

PAC formation between ibuprofen and the ionic polymer has been verified first macroscopically (Figure 1). DLS has been used to monitor both the increase in mean light scattering intensity and the appearance of the autocorrelation curve. The size distribution in intensity of the PACs has been calculated after deconvolution of the autocorrelation curves with the Cumulant method and reveals a mean diameter of PACs in the nanosize range.

**Table 1: Composition of PACs loaded with ibuprofen. Comparison of their macroscopic aspect and of their mean size 2 h after preparation.**

| **Composition and Mw** | **Macroscopic aspect 2h after dissolution** | **Cumulant DLS diameter (nm)** |
|---|---|---|
| | Slightly cloudy | |
| Ibuprofen - PDMAEMA (10 kDa) | No sediment | 380 +/-14 |
| | Slightly cloudy | |
| Ibuprofen PDMAEMA (20 kDa) | No sediment | 381 +/- 6 |
| | Slightly cloudy | |
| Ibuprofen PDMAEMA (90 kDa) | No sediment | 529 +/-17 |
| Ibuprofen alone | Macroscopic crystals | > 1,000,000 |
| PVP K30 (BASF) | Macroscopic crystals | > 1,000,000 |

Surprisingly the results of this experiment (Figure 1 and Table 1) highlight the ability of PDMAEMA to solubilize ibuprofen very quickly at a concentration of at least 5 mg/mL using a drug loading of 10 %. Compared to the known value of maximum solubility of 0.21 µg/mL in water at 25°C, these results show that PDMAEMA increases the solubility of ibuprofen by at least 24,000 times without adding any solvent or surfactant or without using any high thermomechanical energy. Thus, from being practically insoluble, ibuprofen in PACs is now at least slightly soluble.

Being slightly cloudy these nanodispersions are stable and show a mean diameter of PACs loaded with ibuprofen between 380 and 529 nm, depending on the Mw of the ionic polymer. In contrast without any polymer, ibuprofen remains totally insoluble. Keeping in mind the intermediate solubility of PVP, the high dipole present in its repetitive unit and its ability to bind to hydrophobic molecules, this polymer has been tested in the same experimental conditions as PDMAEMA. Although the agitation has been prolonged to 1 day, PVP could not dissolve ibuprofen which remains under the original form of crystals in suspension in water.

It is worth to mention the difference in dissolution kinetics of ibuprofen with the molecular weights (Mw) of ionic polymer. With the lowest Mw of PDMAEMA (10 kDa) it takes only 30 minutes to notice the full dissolution of the drug and polymer powders. In contrast with the highest Mw of PDMAEMA (90 kDa), about 2 hours were required to observe the complete dissolution of the ibuprofen crystal in the aqueous phase. This difference in rate of ibuprofen dissolution could be ascribed to the dissolution rate limitation of the ionic polymer itself, taking into account that polymer chain entanglement is increasing when raising their global length.

The enhancement in solubility of ibuprofen is also visible by the UV spectra of PDMAEMA-ibuprofen which contains 4 peaks between 252 nm and 274 nm (Figure 2) in optical density (Do). Those peaks can be assigned to the aromatic ring present in this molecule. In contrast the ibuprofen dispersed in water does not give any significant signal in this wavelength range. A magnification of this UV domain also clearly attests of the presence of a bathochromic shift (also called red shift) compared to the drug dissolved in methanol. This change is typically assigned to a difference in environmental conditions of the molecule, typically due to a change in solvent polarity, an effect called "solvatochromism". With a red shift, this effect is also called a positive solvatochromism and is frequently noticed when increasing the solvent polarity as is the case when passing from methanol to water. In the far UV domain, i.e. between 245 nm to 210 nm, one main peak (not shown) is well evidenced for free ibuprofen dissolved in methanol or for ibuprofen/PDMAEMA dissolved in water. This peak is assigned to the C=O of the carboxylic group present in this active. All together, these data are therefore confirming that ibuprofen is readily and entirely solubilized in water when physically combined with PDMAEMA.

### EXAMPLE 2: Preparation of Poly Amphiphilic Complexes loaded with ibuprofen by solvent exchange dialysis

As an alternative to example 1, ibuprofen and PDMAEMA have been first dissolved in a binary solvent system made from THF and DMSO (1/1 V/V) to promote dissolution and molecular interaction between the hydrophobic active agent and the polymer.

PACs loaded with ibuprofen are prepared by physical mixing ibuprofen and an ionic polymer made of poly(2-dimethylamino)ethyl methacrylate (PDMAEMA), with a molecular weight of 10 kDa or 20 kDa or 90 kDa. The final concentration of ibuprofen and of PDMAEMA has been fixed to 5 and 45 mg/mL, respectively, to achieve a 10 wt% drug loading. These two compounds are weighed in a Pyrex tube of 5 mL before adding a binary solvent made from THF and DMSO (1/1 V/V). Upon dissolution, the solvents have been extracted by dialysis against water using a semi-permeable membrane having a cut-off of 1 kDa. After purification, the liquid has been freeze-dried.

Particle size of PACs containing curcumin has been analysed by DLS. ¹H.NMR analysis has been conducted on purified and lyophilised ibuprofen-PDMAEMA formulations after their dissolution in CDCI3. The ¹H.NMR spectra shows the protons for ibuprofen and PDMAEMA. Ibuprofen loading has been quantified using the aromatic proton of ibuprofen and the methyl protons linked to the ternary amino group of the repetitive unit of PDMAEMA. As outlined in Table 2, the mean size of the PACs after lyophilisation and microfiltration ranges between 25 and 148 nm.

The results of this experiment attest that the solvent exchange dialysis process also allows promotion of the interaction between PDMAEMA and ibuprofen with an enhancement in its aqueous solubility. Considering a drug loading of 10 wt %, from practically insoluble, ibuprofen is now at least slightly soluble in water.

**Table 2: Ibuprofen loading and mean size of PACs loaded with ibuprofen.**

| **Batch of PAC - Mw of PDMAEMA - Theoretical ibuprofen loading (wt%)** | **Mean size (nm)** | **Ibuprofen loading** (wt%) |
|---|---|---|
| CS009 -10 kDa - 10 % | 25 | 10.8 |
| CS010 - 20 kDa - 10 % | 148 | 9.9 |

### EXAMPLE 3: Preparation of Poly Amphiphilic Complexes loaded with curcumin by direct dissolution in an aqueous phase

Approved by the US Food and Drug Administration (FDA) as "Generally Recognized As Safe" (GRAS), curcumin is widely commercialized as functional food to act as an anti-inflammatory, antioxidant, immunomodulatory, antibacterial, anticarcinogenic, neuroprotective, chemoprotective, vasodilatory, and anti-hyperglycemic agent. However, curcumin is a very hydrophobic active considered as being practically insoluble in water with a solubility estimated to be < 1 µg/mL at 25°C in water and a LogP equal to 3.62.

PACs loaded with curcumin are prepared according to the same method as reported within the example 1 but using curcumin instead of ibuprofen as the hydrophobic active (HA).

Interestingly the presence of PDMAEMA induces a significant change in colour of curcumin which turns from its classical yellow colour to orange (compare the dark coloration on Figures 3A, 3B, and 3C with no coloration in Figures 3D and 3E). This change in the visible absorbance spectra is also correlated to an increase in the concentration of particles in suspension. However, these curcumin formulations remain turbid with presence of sediment. In contrast, free curcumin or curcumin mixed with PVP give rise to slightly yellow and less turbid suspension, the largest fraction of curcumin being settled.

### EXAMPLE 4: Preparation and characterisation of Poly Amphiphilic Complexes loaded with curcumin by solvent exchange dialysis.

The change in optical properties of curcumin formulations detailed on Example 2 shows the existence of molecular interaction between curcumin and PDMAEMA. Alternatively, curcumin and PDMAEMA have been first dissolved in a binary solvent system made from THF and DMSO (1/1 V/V) to promote dissolution and molecular interaction between the active and the polymer.

PACs loaded with curcumin are prepared by physical mixing curcumin and an ionic polymer made of poly(2-dimethylamino)ethyl methacrylate (PDMAEMA), with a molecular weight of 10 kDa or 20 kDa. The final concentration of curcumin and of PDMAEMA has been fixed to 5 and 45 mg/mL, respectively, to achieve a 10 % drug loading. These two compounds are weighed in a Pyrex tube of 5 mL before adding a binary solvent made from THF and DMSO (1/1 V/V). Upon dissolution, the solvents have been extracted by dialysis against water using a semi-permeable membrane having a cut-off of 1 kDa. After purification, the liquid has been freeze-dried. A control has been made adopting the same procedure but using a di-block copolymer made of polyethylene oxide -b- poly(d,l)-lactide (5 kDa-5 kDa) (PEO-P(d,l)LA).

As already noticed in Example 2, curcumin associated to PDMAEMA gives rise to a change in visible absorbance spectra, turning here to a brown dark solution (see Figures 4A (CS001) and 4B (CS002) in comparison to Figure 4C (CS005)). In contrast to Example 2, no sediment was observed in the formulation either before or after lyophilisation. In contrast, the formulation of curcumin mixed with PEO-P(d,l)LA shows a slightly yellow aspect. These changes in optical density of curcumin solution is confirmed by their visible spectra given on Figure 5 which shows both an increase in optical density (Do) and a red shift when curcumin is associated to PDMAEMA.

Particle size of PACs containing curcumin has been analysed by DLS. ¹H.NMR analysis has been conducted on purified and lyophilised curcumin-PDMAEMA formulations after their dissolution in CDCl₃. The ¹H.NMR spectra shows the protons for curcumin and PDMAEMA. Curcumin loading has been quantified using the methyl proton of methoxy groups of curcumin and the methyl protons linked to the ternary amino group of the repetitive unit of PDMAEMA. As outlined in Table 3, the mean size of the PACs after lyophilisation and microfiltration is not significantly affected by the drug content no more by the Mw of the ionic polymer and ranges between 0 and 35 nm.

The results of this experiment attest that the solvent exchange dialysis process allows also to promote the interaction between PDMAEMA and curcumin with an enhancement in its aqueous solubility. Using a drug loading of 30 wt %, from practically insoluble, curcumin is now at least sparingly soluble in water.

**Table 3: Curcumin loading and mean size of PACs loaded with curcumin.**

| **Batch of PAC - Mw of PDMAEMA - Theoretical curcumin loading (wt%)** | **Mean size (nm)** | **Curcumin loading (wt%)** |
|---|---|---|
| CS021 -10 kDa - 10 % | no DLS curve visible | 12.27 |
| CS022 - 20 kDa - 10 % | 35 | 8.80 |
| CS023 - 10 kDa - 20 % | no DLS curve visible | 17.35 |
| CS024 - 20 kDa - 20 % | no DLS curve visible | 16.74 |

**Table 4: Evolution with time of the optical density (Do at 405 nm) of solutions made from free curcumin or PDMAEMA - curcumin. These solutions were buffered in a PBS at pH 7.4 and stored 4 days at 37°C.**

| **Free curcumin** | | | | | **PDMAEMA curcumin** | | | | |
|---|---|---|---|---|---|---|---|---|---|
| **Condition** | **Curcumin (µg/mL)** | **Duration (h)** | | | **Condition** | **Curcumin (µg/mL)** | **Duration (h)** | | |
| | | **1** | **24** | **96** | | | **1** | **24** | **96** |
| Without BSA | 2 | 0.068 | 0.019 | 0.001 | Without BSA | 2 | 0.066 | 0.076 | 0.110 |
| | 10 | 0.321 | 0.097 | 0.064 | | 10 | 0.533 | 0.271 | 0.211 |
| | 20 | 0.717 | 0.253 | 0.113 | | 20 | 1.263 | 0.710 | 0.386 |
| With BSA (1%) | 2 | 0.275 | 0.214 | 0.437 | With BSA (1%) | 2 | 0.237 | 0.249 | 0.375 |
| | 10 | 1.102 | 1.007 | 0.701 | | 10 | 0.718 | 0.644 | 0.611 |
| | 20 | 1.875 | 1.508 | 0.899 | | 20 | 1.695 | 1.492 | 1.299 |

The evolution of the optical density (Do at 405 nm) of solutions made from free curcumin or PDMAEMA-curcumin has been monitored with time. These solutions of curcumin have been buffered in PBS at pH 7.4 and stored up to 4 days at 37°C. Free curcumin solutions in PBS have been prepared starting from a stock solution in DMSO (1 mg/mL). The results summarized in Table 4 clearly highlight the important instability of curcumin with a very rapid decrease in Do. This observation is not surprising taking into account that its solubility limit has been reported to be < 1 µg/mL in aqueous medium. The procedure to prepare solution of curcumin in the µg/mL concentration range by a dilution from a stock solution in DMSO, gives rise to sur-saturated solution which are not stable in aqueous buffers. DLS of these free curcumin solutions has evidenced the rapid aggregation of curcumin when free in solution which explains the decrease in optical density.

In the presence of Bovine Serum Albumin (BSA) at a concentration of 10mg/mL, curcumin is more stable and is associated with an absorption enhancement in the visible range and to a red-shift. These observations are in line with former results reported by SANKAR et al. [J. Surface Sci. Technol., 2007, Vol 23, (3-4), 91-110, Binding and Stability of Curcumin in Presence of Bovine Serum Albumin]. These authors have indeed shown that curcumin has a strong affinity to this protein with an affinity constant Ka of ∼ 6.3 x 10⁻⁶ M, but with a moderate loading capacity (n ∼ 0.30 Mole of curcumin / Mole of BSA). In terms of weight, this loading capacity means that albumin can bind 0.17 wt % of curcumin relative to its weight. Within a 10 mg/mL of BSA solution, we can therefore readily calculate that it could be possible to dissolve a maximum concentration of curcumin of 17 µg/mL.

The incubation of PACs loaded with curcumin in the presence of BSA also enhances the optical density (Do) of curcumin in the visible domain. Their Do evolution with time highlights a higher stability of curcumin, with only a Do decrease of 25% on a 4 day-period of incubation. Accordingly we can anticipate the existence of two physicochemical events which could occur upon mixing PACs loaded with curcumin in the presence of BSA: i) polyelectrolyte complexation formation between PDMAEMA and BSA; ii) migration of curcumin from PDMAEMA to BSA, as a function of the diffusion rate and difference in the affinity of curcumin for these two macromolecules. A progressive transfer of curcumin from the ionic polymer (PDMAEMA) to plasma protein (BSA) would be particularly valuable in order to guarantee the bioavailability and pharmacological activity of the drug.

Incubation of curcumin/PDMAEMA formulation at acidic pH has also evidenced a rapid dissociation of curcumin. Indeed, upon incubation of this formulation at pH 2.5, it turns immediately from brown to yellow with the appearance of large yellow crystals made from curcumin. Without wishing to be bound by any particular theory, considering the respective pKa values of curcumin and PDMAEMA (i.e. 7.8; 8.5 and 9.0 for curcumin and 6.7 for PDMAEMA), one main driving force of this interaction could rely upon hydrophobic and ionic interactions. Indeed, ionic interactions can be expected between this active and our polycation in a range of pH where this polycation bears positive charges (i.e. below pH 8.4) and above pH 7.0 where the first phenolic function of curcumin can lose its proton. Hydrophobic interactions between curcumin and PDMAEMA may be enhanced thanks to the progressive deprotonation of its ternary amino groups, being mostly hydrophobic and insoluble in aqueous medium above pH 8.5.

The abilityto modulate these interactions in a range of pH close to the physiological environment is useful. In particular, this rapid release of curcumin upon acidification could trigger the release of this drug just upon cell internalization through endocytosis. Indeed, if this pathway is used to internalize the PDMAEMA-curcumin complex within cells, the local acidic pH within endolysomes (around pH 5 to 6) could trigger the release of curcumin from PDMAEMA.

### EXAMPLE 5: In vitro antimalarial activity assays of PDMAEMA - curcumin formulations

Since P. *falciparum* I usually asynchronous during in vitro culture, the generation of highly synchronized parasite cultures is requested to investigate their pharmacological properties in vitro. For in vitro drug assays, a % of at least 70 of ring stage are typically advised (Exp Parasitol. 2014; 140: 18-23). PACs loaded with curcumin were analysed with 3D7 cells synchronized at ring stage. Samples of the PACs were incubated with the cells for 48 h and then parasitemia was evaluated by flow cytometry after labelling parasite nuclei with fluorescence (growth inhibition assay, GIA). The pharmacological activity of curcumin is expressed in terms of drug concentration requested to inhibit 50% of the cell population (IC50) (see Table 5). Compared to free curcumin first dissolved in DMSO and diluted afterwards in cell culture medium containing proteins, all four formulations of curcumin-PDMAEMA batches listed in Table 5 have provided a lower IC50than free curcumin (14.23 µM). These *in vitro* data are therefore demonstrating that curcumin associated to the ionic polymer is pharmacologically effective, and still slightly more potent than the free drug first dissolved in a solvent before diluting it in a medium where it can be associated with proteins.

**Table 5: In vitro antimalarial activity assays of PDMAEMA - curcumin formulations using the growth inhibition assay, GIA. The pharmacological activity of curcumin is expressed in terms of drug concentration requested to inhibit 50 % of the cell population (IC50)**

| **Sample nature** | **Curcumin loading (%)** | **IC50 (µM)** |
|---|---|---|
| Free curcumin dissolved in DMSO | - | 14.23 |
| CS021- PDMAEMA 10 kDa | 12.27 | 11.26 |
| CS022 - PDMAEMA 20 kDa | 8.80 | 9.02 |
| CS023 - PDMAEMA 10 kDa | 17.35 | 12.80 |
| CS024 - PDMAEMA 20 kDa | 16.74 | 10.44 |

### COMPARATIVE EXAMPLE 6: Preparation of Eudragit or PVP formulations loaded with curcumin by solvent exchange dialysis.

Various copolymers known as Eudragits (L100, S100, RL100, RS100, EPO or L100-55) and polyvinyl pyrrolidone PVP K30 (BASF) were loaded with curcumin by physically mixing this hydrophobic active (HA) and these polymers according to the procedure explained in Example 2. The concentration of curcumin and of the polymers has been fixed to 5 and 45 mg/mL to achieve a 10 % drug loading. After dialysis, the different liquids have been freeze-dried. The dissolution ability of curcumin by these different polymers has been assessed according to the macroscopic aspect of the freeze-dried powders redispersed in a saline medium buffered at pH 2.5, 7.4 or 8.5.

None of the different Eudragit commercial polymer excipients give rise to stable and homogeneous formulations at neutral pH or in slightly alkaline medium. At pH 2.5 yellow solutions have been noticed with PVP K30 (BASF) and Eudragit EPO.

### EXAMPLE 7: Preparation of Poly Amphiphilic Complexes loaded with atovaquone by solvent exchange dialysis

Atovaquone is a synthetic hydroxyl-naphthoquinone with antiprotozoal activity. Atovoquone blocks the mitochondrial electron transport at complex III of the respiratory chain of protozoa, leading to protozoal death. Atovaquone is very hydrophobic with a LogP 5.8 and is therefore practically insoluble in water. In vivo this drug is extensively bound to plasma proteins (99.9%).

According to the procedure of Example 2, PACs loaded with atovaquone are prepared by physically mixing this hydrophobic active agent with an ionic polymer made of poly(2-dimethylamino)ethyl methacrylate (PDMAEMA), with a molecular weight of 10 kDa, 20 kDa or 90 kDa. The final concentration of atovaquone and of PDMAEMA has been fixed to 5 and 45 mg/mL to achieve a 10 % drug loading.

After dialysis, the different liquids have been freeze-dried. The solubilisation has been assessed macroscopically after reconstitution of the freeze-dried powders in a saline medium buffered at 7.4. As observed in Figures 6A, 6B, and 6C, the atovaquone formulations are totally transparent and have a light color (slightly orange). Atovaquone loading has been analysed by NMR in CDCl₃. The ¹H.NMR spectra show the protons of atovaquone and PDMAEMA. Atovaquone loading has been quantified using aromatic protons of atovaquone and the methyl protons linked to the ternary amino group of the repetitive unit of PDMAEMA. Compared to the theoretical drug loading (10 %), atovaquone content ranges between 7.6 and 8.7 %, thus increases slightly with the Mw of PDMAEMA (Table 6).

Particle size of PACs loaded with atovaquone has been analysed by DLS. The mean size of the PACs is below 10 nm for the batches prepared with PDMAEMA of a Mw 10 kDa and 20 kDa and is about 117 nm for PAC made of PDMAEMA having a Mw of 90 kDa.

**Table 6: Atovaquone content in PACs made from PDMAEMA according to Example 6 and mean size of the atovaquone formulation after their freeze-drying and after reconstitution of the freeze-dried powders in a saline medium buffered at pH7.4 (No DLS autocorrelation curve means that no aggregate having a size greater than 1 nm is detected).**

| **Batch** | **Mw PDMAEMA** | **Atovaquone (wt%)** | **Mean size (nm)** |
|---|---|---|---|
| CS041 | 10 | 7.6 | 5 +/- 8 |
| CS042 | 20 | 7.8 | No DLS curve visible |
| CS043 | 90 | 8.7 | 117+/- 3 |

Solubilisation of atovaquone in water is also demonstrated by the UV-visible spectra of the PACs redissolved in water before and after proceeding to their microfiltration (0.2 µm) as disclosed in Figure 7. Whatever the Mw of the ionic polymer (10 to 90 kDa), the absorbance of atovaquone is well detected both in the close UV and visible spectra. The reduction in optical density (Do) noticed in the whole spectrometric spectra after filtration could be assigned to a partial adsorption of the PACs loaded with atovaquone to the surface of the polysulfone filter.

### EXAMPLE 8: Monitoring of the visible spectra of PACs of atovaquone (10wt%) - PDMAEMA as a function of time, pH and the presence or absence of BSA (1 wt/vol%)

As disclosed in Example 6, we have monitored the possible change in UV-visible spectra of atovaquone associated with the ionic polymer (PDMAEMA) compared to the free drug. In this study we have assessed the influence of the following experimental variables: i) the pH of a phosphate buffer: pH 2.5; 7.4 or 8.5; ii) the Mw of the ionic polymer: 10; 20 or 90 kDa; and iii) the presence or absence of bovine serum albumin (1 wt/vol%).

Free atovaquone, first dissolved in DMSO (1 mg/mL) and diluted 10 times in phosphate buffer, leads to yellow solutions which are slightly turbid and unstable whatever the pH of the buffer. This turbidity explains the large tailing peak which is covering the entire visible spectra (Figure 8A). If BSA (10 mg/mL) is added in the phosphate buffer, the free atovaquone solutions turn from yellow to orange at pH 7.4 and 8.5 while being still more cloudy and yellow at pH 2.5. Those changes are indicated by the presence of a significant absorption peak of atovaquone between 492 nm and 498 nm (Figure 8B).

As already noticed for curcumin, the color of the atovaquone-PDMAEMA solutions is a function of the pH of the buffer. At acidic pH they are yellow whatever the Mw of the ionic polymer, while being orange/red at neutral or slightly alkaline pH. Accordingly, their visible spectra changes as a function of the pH, with a relatively wide peak noticed between 400 to 450 nm at pH 2.5 and a narrow peak at pH 7.4 and 8.5 (Figure 8C).

When PACs loaded with atovaquone are dissolved in a phosphate buffer containing BSA (1wt/vol%), the Do peak at 495 nm is also well evidenced and shows a profile similar to the Do peak observed without BSA at both pH: 7.4 and 8.5 (Figure 8D). At acidic pH, the visible spectrum is indicative of the turbidity and instability of atovaquone as already noticed for the free drug.

All these spectroscopic and macroscopic observations are therefore demonstrating the existence of a strong molecular interaction existing between atovaquone and the ionic polymer; an interaction which is affected by the pH and the presence of proteins.

The results of this experiment are also attesting that the solvent exchange dialysis process allows to promote the interaction between PDMAEMA and atovaquone with an enhancement in its aqueous solubility. Using a drug loading of 10 wt %., from practically insoluble, atovaquone is now at least slightly soluble in water.

### EXAMPLE 9: In vitro antimalarial activity assay of PDMAEMA - atovaquone formulations

PACs loaded with atovaquone were analysed with 3D7 cells synchronized at ring stage. Samples were incubated with the cells for 48 h and then parasitemia was evaluated by flow cytometry after labelling parasite nuclei with fluorescence (growth inhibition assay, GIA). The pharmacological activity of atovaquone is expressed in terms of drug concentration needed to inhibit 50 % of the cell population (IC50) (Figure 9). Free atovaquone first dissolved in DMSO and diluted afterwards in cell culture medium containing proteins has a IC50 equal to 0.84 nM. By comparison, the three formulations of atovaquone-PDMAEMA batches have a IC50 12 to 24 times higher. Although with a lower pharmacological potency than the free drug, these *in vitro* data are demonstrating that atovaquone associated to the ionic polymers is pharmacologically effective. The higher activity of the free drug could be explained by a slow release rate of atovaquone from the ionic polymer and the *in vitro* conditions used to carry out this assay.

### EXAMPLE 10: In vivo determination of the anti-parasite effect by feeding mosquitoes with PAC's loaded with atovaquone

Transmission blocking has been tested by feeding female *Anopheles gambiae* mosquitoes with PACs loaded with atovaquone dissolved in 10 wt/vol% sucrose solution then allowing the mosquitoes to feed on a mouse infected with GFP-expressing parasites. Two cups were set up with 40 mosquitoes each. A control cup was fed with sucrose and a test cup with 1 µM atovaquone loaded within PAC CS090 (PDMAEMA 10kDa, 10wt% loaded with atovaquone). This feeding lasted 1 day. On the second day, mosquitoes were starved for two hours and allowed to feed on a mouse infected by *Plasmodium berghei* (Bergreen strain).

After the blood feeding, mosquitoes were allowed to feed on CS090 until dissection.

Six days post infection (dpi) after blood feeding the mosquitoes were dissected and the oocysts on the midguts were counted under the microscope.

In the control group, nearly all mosquitoes got infected (96%) while in the group test having received PAC made from PDMAEMA (10 kDa) and atovaquone, 66% of mosquitoes were infected (difference is significant P<0.05, Mann-Whitney). There was no difference in mean number of oocysts/midgut in the two groups (control 14, test 13). For some mosquitoes, the parasite is not affected at all by atovaquone. This may mean that in some mosquitoes atovaquone does not reach the midgut, is not released from the PAC, or is rapidly metabolised/excreted.

### EXAMPLE 11: Preparation of Poly Amphiphilic Complexes loaded with ivermectin or ticagrelor by solvent exchange dialysis

Ivermectin is an FDA-approved broad spectrum anti-parasitic agent that in recent years have shown to have anti-viral activity against a broad range of viruses (including HIV and more recently SARS-CoV-2). Ticagrelor is an oral antiplatelet drug that is used with low dose aspirin to decrease the risk of myocardial infarction and stroke in patients with acute coronary syndromes. More recently ticagrelor has been proposed also as a new antibiotic against resistant Gram-positive bacteria. These two drugs are hydrophobic compounds with LogP equal to 2.28 and 5.83 for ticagrelor and ivermectin respectively.

According to the methodology of Example 2, PACs loaded with each of these two actives are prepared by physically mixing each of these hydrophobic actives (HA) with an ionic polymer made of poly(2-dimethylamino)ethyl methacrylate (PDMAEMA), with a molecular weight of either 10 kDa or 90 kDa (for Ivermectin). The final concentration of each of these two drugs and of PDMAEMA has been fixed to 5 and 45 mg/mL to achieve a 10wt/vol% drug loading. After dialysis, the different liquids obtained have been freeze-dried. The dissolution ability of these drug formulations has been observed macroscopically after reconstitution of the freeze-dried powders in a saline medium buffered at pH7.4. PACs loaded with ticagrelor give rise to transparent and stable solutions, while the dispersions of PACs loaded with ivermectin are stable but slightly cloudy. This difference in macroscopic properties of these formulations is confirmed by the analysis of the particle size determined by DLS. As outlined in Table 7, the mean size of the PACs is below 10 nm for the formulations loaded with ticagrelor, whatever the Mw of PDMAEMA and is around 300 nm for PACs loaded with ivermectin.

HA loadings have been analysed by NMR in CDCl₃ using their aromatic protons and the methyl protons linked to the ternary amino group of the repetitive unit of PDMAEMA. Compared to the theroretical drug loading (10wt/vol%), the HA content ranges between 9.4 and 15.1wt/vol%, thus increases slightly with the Mw of PDMAEMA (Table 7).

The results of this experiment attest that the solvent exchange dialysis process allows to promote the interaction between PDMAEMA and ivermerctin or ticagrelor with an enhancement in their aqueous solubility. Using a drug loading of 10 wt %., from practically insoluble, ivermerctin and ticagrelor are now at least slightly soluble in water.

**Table 7: HA content in PACs made from PDMAEMA according to Example 10 and mean size of the HA formulations after their freeze-drying and after reconstitution of the freeze-dried powders in a saline medium buffered at 7.4**

| **Batch** | **HA drug** | **Mw PDMAEMA (kDa)** | **Mean size (nm)** | **Drug content (%)** |
|---|---|---|---|---|
| CS191 | Ticagrelor | 10 | 3 | 15.1 |
| CS197 | Ivermectin | 10 | 325 | 9.4 |
| CS198 | Ivermectin | 90 | 300 | 11.2 |

### EXAMPLE 12: Surface modification of Poly Amphiphilic Complexes loaded either with curcumin or atovaquone

Surface modification of drug carriers, such as liposomes, polymeric nanoparticles has been reported to reduce their clearance and promote their cellular uptake. Heparin, a hydrophilic polysaccharide is able to provide a steric barrier but can also promote cellular internalisation of the nanovehicles. Accordingly, heparin has been adsorbed to the surface of poly amphiphilic complexes formed from PDMAEMA having a Mw of 10kDa, 20kDa, or 90kDa loaded either with curcumin or atovaquone whose preparation has been disclosed in Example 4 and 7 respectively. 10 mg of these PACs have been dissolved in 16 mL of a phosphate medium buffered at pH 7.4. After 1h of dissolution carried out at room temperature, a solution of heparin from porcine origin has been added to the PACsolution in order to achieve a final concentration of heparin between 0.0005wt/vol% and 0.05wt/vol%. One hour after equilibration, the formulations have been filtrated on 0.2 µm filter, freeze-dried and resuspended in water for particle size analysis by DLS. As disclosed in Table 8, the mean size of the PACs after heparin coating and freeze-drying remains in the nanometer range when the heparin concentration remains below a concentration threshold in the coating medium. Above a heparin concentration of 0.05wt/vol%, PAC aggregation has been noticed.

**Table 8: Mean size of the PAC's made from PDMAEMA and loaded with curcumin or atovaquone after modification of their surface with heparin according to conditions given in Example 11.**

| **Batch** | **HA drug and wt/vol%** | **Mw of PDMAEMA (kDa)** | **Mean PAC size (nm)** |
|---|---|---|---|
| CS081F | Curcumin -10 % | 10 | -* |
| CS082F | Curcumin -10 % | 20 | 10 |
| CS083F | Atovaquone -10 % | 10 | 84 |
| CS084F | Atovaquone -10 % | 20 | 111 |
| CS085F | Atovaquone -10 % | 90 | 25 |

| | | | |
|---|---|---|---|
| * no correlation curve has been observed | | | |

### EXAMPLE 13: Preparation of Poly Amphiphilic Complexes loaded with hydrophobic peptide by solvent exchange dialysis

Clinical application of hydrophobic peptide therapeutics, such as self-assembling peptides, is restricted by a lack of efficient vehicles allowing their transport within biological fluids. PACs loaded with a hydrophobic peptide and an ionic polymer were prepared according to the methodology of Example 2 by solvent exchange dialysis. The molecular weight of each peptide, their isoelectric point (pi) and their solubility are given on Table 9. Each peptide has been loaded in an ionic polymer PDMAEMA (20 kDa) with a drug content of 1wt/vol%.

After purification and lyophilisation, the mean size of the PAC has been measured by DLS. Homogenous and stable dispersion of PAC has been noticed for five of the seven hydrophobic peptides. The mean size of their dispersion in water range between 2381 and 4137 nm (see Table 9). For the peptide nos. 4 and 7, no solubility enhancement has been noticed.

The results of this experiment attest that the solvent exchange dialysis process allows to promote the interaction between PDMAEMA and the hydrophobic peptides with an enhancement in their aqueous solubility. Using a drug loading of 1 wt %., from practically insoluble, these peptides are now at least very slightly soluble in water.

**Table 9: Sequence, pl and solubility of aggregative peptides which have been loaded within PAC's. The mean size of these PAC's prepared according to example 13 after purification and lyophilisation have been measured by DLS.**

| **Batch of PAC** | **Peptide No** | **Mw** | **pl** | **Solubility (µg/mL)** | **Mean size (nm)** |
|---|---|---|---|---|---|
| CS093 | 1 | 705 | 5.59 | < 100 | 2381 |
| CS094 | 2 | 787 | 5.33 | < 100 | 4137 |
| CS095 | 3 | 920 | 5.53 | < 100 | 3220 |
| CS096 | 4 | 720 | 5.33 | < 100 | - |
| CS097 | 5 | 695 | 5.61 | < 100 | 2654 |
| CS098 | 6 | 789 | 5.22 | < 100 | 4070 |
| CS099 | 7 | 631 | 5.58 | < 100 | - |

## Claims

1. A complex comprising at least one hydrophobic active agent and an ionic polymer comprising a repetitive unit of formula (I): 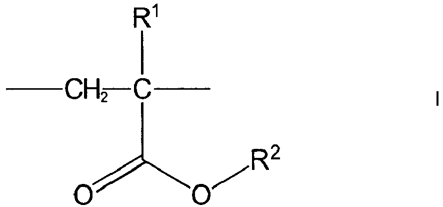
wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; with the proviso that where the ionic polymer comprising a repetitive unit of formula (I) is poly(2-dimethylamino)ethyl methacrylate having an ionisation percentage of 30% and a molecular weight of 7, 13, 26.4, 49.1, or 91.4 kDa, the hydrophobic active agent is not ovalbumin;
with the proviso that where the ionic polymer comprising a repetitive unit of formula (I) is poly(2-dimethylamino)ethyl methacrylate having a molecular weight of 13 kDa and an ionisation percentage of 30%, 47%, 60%, or 80%, the hydrophobic active agent is not ovalbumin or human insulin;
with the proviso that where the ionic polymer comprising a repetitive unit of formula (I) is poly(2-dimethylamino)ethyl methacrylate having a molecular weight of 10 or 90 kDa, the hydrophobic active agent is not ovalbumin;
with the proviso that where the ionic polymer comprising a repetitive unit of formula (I) is poly(2-dimethylamino)ethyl methacrylate having an ionisation percentage of 30% and a molecular weight of 13, 14, 26.4, 49.1, 90, or 91.4 kDa, the hydrophobic active agent is not human insulin;
with the proviso that where the ionic polymer comprising a repetitive unit of formula (I) is a copolymer of poly(2-dimethylamino)ethyl methacrylate having a molecular weight of 7 kDa and poly(ethylene oxide) having a molecular weight of 0.5 kDa, the hydrophobic active agent is not human insulin;
with the proviso that where the ionic polymer comprising a repetitive unit of formula (I) is poly(2-dimethylamino)ethyl methacrylate having a molecular weight of 10, 20,40, or 90 kDa and the hydrophobic active agent is human insulin, the complex does not contain sodium dodecyl sulphate;
with the proviso that where the ionic polymer comprising a repetitive unit of formula (I) is poly(2-dimethylamino)ethyl methacrylate having a molecular weight of 10 or 90 kDa and the hydrophobic active agents are P24 structural protein of HIV viral capside and CpG short single-stranded synthetic DNA molecule, the complex does not contain sodium dodecyl sulphate, Tween 20, C8S 04, C11COO-, or PE6800.

2. A complex comprising at least one hydrophobic active agent and an ionic polymer comprising a repetitive unit of formula (I): 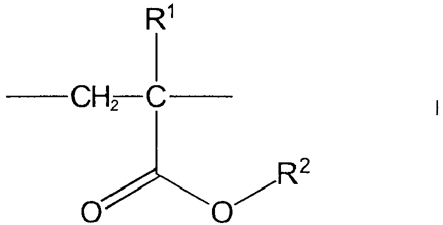 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; wherein the at least one hydrophobic active agent has a molecular weight of from 100 to 1500 g/mol; preferably the at least one hydrophobic active agent has a molecular weight of from 100 to 1000 g/mol.

3. A complex as defined in Claim 1 or Claim 2 wherein the ionic polymer comprises a single repetitive unit of formula (I).

4. A complex as defined in Claim 1 or Claim 2 wherein the ionic polymer comprises a repetitive unit of formula (I) in combination with one or more other monomers, other polymers, and/or oligomeric sequences.

5. A complex as defined in any one of the preceding Claims wherein the ionic polymer comprises repetitive units of formula (I) wherein from 10% to 70% of the repetitive units of formula (I) are charged.

6. A complex as defined in any one of the preceding Claims wherein the ionic polymer has a mean pKa of from 6 to 8.

7. A complex as defined in any one of the preceding Claims wherein the repetitive unit of formula (I) comprises N,N dimethyl amino ethyl methacrylate.

8. A complex as defined in any one of the preceding Claims wherein the molar ratio of (dimethyl amino) ethyl methacrylate to a different repetitive unit of formula (I) is greater than 50%; preferably the molar ratio of (dimethyl amino) ethyl methacrylate to a different repetitive unit of formula (I) wherein R¹ represents a straight or branched chain alkyl group comprising from 1 to 6 carbon atoms is greater than 50%.

9. A complex as defined in any one of the preceding Claims wherein the hydrophobic active agent is sparingly soluble, slightly soluble, very slightly soluble or practically insoluble.

10. A complex as defined in any one of the preceding Claims which has an aqueous concentration of from 1 mg/ml to 200 mg/ml; preferably from 10 mg/ml to 100 mg/ml.

11. A complex as defined in any one of the preceding Claims wherein the hydrophobic active agent has a LogP value which is from 2 to 8.

12. A complex as defined in any one of the preceding Claims which has a dynamic light scattering size of from 1 to 5000 nm.

13. A complex as defined in any one of the preceding Claims which has a surface modification; preferably the surface modification is with an anionic hydrosoluble molecule; more preferably the surface modification is with heparin.

14. A complex for use as a medicament wherein the complex comprises at least one medically active hydrophobic active agent, an ionic polymer comprising a repetitive unit of formula (I): 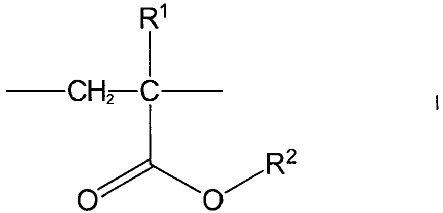 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; or a copolymer thereof; preferably the complex is as defined in any one of claims 2 to 13.

15. A pharmaceutical composition comprising a pharmaceutically acceptable carrier and a complex as defined in any one of claims 1 to 13.

16. A pharmaceutical composition for use as a medicament wherein the composition comprises a pharmaceutically acceptable carrier and a complex as defined in claim 14; preferably the complex is as defined in any one of claims 3 to 13.

17. A method of medical treatment which method includes a step of administering to a human or animal in need of such treatment an effective amount of a complex which comprises at least one medically active hydrophobic active agent, an ionic polymer comprising a repetitive unit of formula (I) as defined in Claim 14 or a copolymer thereof; and optionally a surfactant; preferably the complex is as defined in any one of claims 2 to 13.

18. A method of preparing a complex as defined in Claim 14 which method comprises the steps of:
(a) dissolving the hydrophobic active agent and the ionic polymer in one or more non-aqueous solvents to form the complex wherein the one or more non-aqueous solvents are miscible with water; and
(b) progressively replacing the one or more non-aqueous solvents with water.
